(19) Europäisches Patentamt
European Patent Office
Office européen des brevets

(11) Publication number: 0 403 938 A2

# (12) EUROPEAN PATENT APPLICATION

(21) Application number: 90111120.3

(51) Int. Cl.5: C07D 307/20, A01N 43/08

(22) Date of filing: 12.06.90

(30) Priority: 15.06.89 JP 152595/89
31.05.90 JP 141877/90

(43) Date of publication of application:
27.12.90 Bulletin 90/52

(84) Designated Contracting States:
CH DE FR GB IT LI

(71) Applicant: MITSUBISHI KASEI CORPORATION
5-2, Marunouchi 2-chome Chiyoda-ku
Tokyo 100(JP)

(72) Inventor: Jikihara, Tetsuo
3-14-3-103 Higashi-Izumi
Komae-shi, Tokyo(JP)

Inventor: Watanabe, Hisao
3-3-224, Tsutsujigaoka Midori-ku,
Yokohama-shi, Kanagawa-ken(JP)
Inventor: Suzuki, Seiichi
2-32-4-21-306 Narusedai
Machida-shi, Tokyo(JP)
Inventor: Minami, Noriko
5-10-12 Seijo, Setagaya-ku
Tokyo(JP)

(74) Representative: TER MEER - MÜLLER - STEINMEISTER & PARTNER
Mauerkircherstrasse 45
D-8000 München 80(DE)

## (54) Diphenyl ether derivative, herbicidal composition containing the derivative as active ingredient, and process for producing the derivative.

(57) This invention provides a diphenyl ether derivative represented by the formula (I):

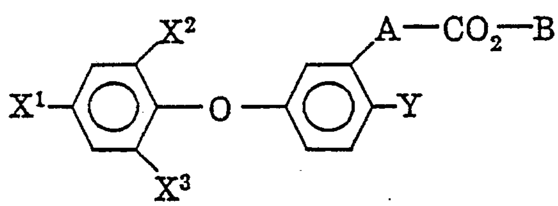

(I)

wherein $X^1$ represents a halogen atom or a $C_1$-$C_3$ haloalkyl group; $X^2$ and $X^3$ represent independently a hydrogen atom, a halogen atom, a trifluoromethyl group, a nitro group or a cyano group; Y represents a nitro group, a cyano group, a halogen atom, a $C_1$-$C_3$ haloalkyl group or a hydrogen atom; A represents a direct bond, a group represented by the formula:

$-CO_2(CH(R^1)-$

wherein $R^1$ represents a hydrogen atom or a $C_1$-$C_3$ alkyl group, a group represented by the formula:

$-C(R^2) = NOCH(R^3)-$

wherein $R^2$ represents a hydrogen atom, a $C_1$-$C_4$ alkyl group which may be substituted with a $C_1$-$C_4$ alkoxy group, $C_1$-$C_4$ alkylthio group or halogen atom, or a $C_1$-$C_3$ alkoxy group, and $R^3$ represents a hydrogen atom or a $C_1$-$C_3$ alkyl group, or a group represented by the formula:

$-C(=NOR^4)OCH(R^3)-$

wherein $R^3$ is the same as defined above and $R^4$ represents a $C_1$-$C_3$ alkyl group; and B represents a group represented by the formula:

EP 0 403 938 A2

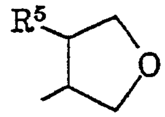

wherein $R^5$ represents a halogen atom; a hydroxyl group; an acyloxy group having up to 6 carbon atoms which may be substituted with a halogen atom, $C_1$-$C_6$ alkoxy group, $C_2$-$C_7$ alkoxycarbonyl group, aryl group which may be substituted with 1 to 3 halogen atoms and/or 1 to 3 $C_1$-$C_3$ alkyl groups or aryloxy group which may be substituted with 1 to 3 halogen atoms and/or 1 to 3 $C_1$-$C_3$ alkyl groups; a $C_4$-$C_9$ cycloalkylcarbonyloxy group; a benzoyloxy group which may be substituted with a halogen atom, $C_1$-$C_4$ alkyl group or $C_1$-$C_4$ alkoxy group; a $C_2$-$C_7$ alkoxycarbonyloxy group; a $C_1$-$C_4$ alkylsufonyloxy group; or a carbamoyloxy group which may be substituted with a $C_1$-$C_4$ alkyl group and/or $C_1$-$C_4$ alkoxy group. A process for preparing the diphenyl ether derivative and a herbicidal composition containing the diphenyl ether derivative as active ingredient are also described. The compounds of this invention show a wide herbicidal spectrum against both annual and perennial weeds and also have a high degree of safety or harmlessness to crops.

# DIPHENYL ETHER DERIVATIVE, HERBICIDAL COMPOSITION CONTAINING THE DERIVATIVE AS ACTIVE INGREDIENT, AND PROCESS FOR PRODUCING THE DERIVATIVE

The present invention relates to a novel diphenyl ether derivative and a herbicidal composition containing the derivative as active ingredient.

A large number of diphenyl ether compounds having a herbicidal activity, with 2,4-dichloro-4′-nitrodiphenyl ether (generally called nitrofen) as the first instance, have been reported. Various kinds of herbicide compounds have been obtained by the modification at the 4-position and 3′-position (ortho position with respect to the nitro group in nitrofen). Typical examples of such herbicides are 2,4-dichloro-3′-methoxycarbonyl-4′-nitrodiphenyl ether (common name: bifenox), sodium 5-(2-chloro-4-trifluoromethyl-phenoxy)-2-nitrobenozate (common name: acifluorfen-sodium), 1-(ethoxycarbonyl)ethyl 5-(2-chloro-4-trifluoromethylphenoxy)-2-nitrobenzoate (common name: lactofen), and 5-(2-chloro-4-trifuoromethylphenoxy)-2-nitro-N-methanesulfonylbenzamide (common name: fomesafen). Also, diphenyl ethers having tetrahydrofuran rings are disclosed in Japanese Patent Application Laid-Open(KOKAI) Nos. 56-86179(1981), 56-108742(1981) and 60-1156(1985). Further, use of (substituted) alkyl (5-(2-chloro-4-trifluromethylphenoxy)phenyl) ketone derivatives as a herbicide is proposed in Japanese Patent Application Laid-Open (KOKAI) Nos. 56-32432(1981), 60-226856(1985), etc. and the use of 2-nitro-5-(substituted phenoxy)benzohydroximic acid derivatives as a herbicide is reported in Japanese Patent Application Laid-Open (KOKAI) Nos. 62-201860(1987) and 63-165359(1988).

However, the crop which shows resistance to the phytotoxicity due to the conventional diphenyl ether herbicides is limited to only certain specific crop such as rice plant, soybean and the like. This is for the reason that the conventional diphenyl ether herbicidal compounds tend to be low in safety to crops when their herbicidal activity is very high, while they tend to be low in herbicidal activity when their safety to crops is high, and thus none of the conventional diphenyl ether herbicides shows high activity and high selectivity to many other important crops, at the same time.

As stated above, although many diphenyl ether compounds having herbicidal activity have been proposed, there still are few which can satisfy both high safety to crops and satisfactory herbicidal activity.

Therefore, a herbicide satisfying both requirements has been desired.

It is needless to say that a herbicidal compound capable of bringing about the desired effect with a minimum amount of application is preferred, considering influence to the environment.

In the present inventors' research to give a solution to the above problem, the present inventors have found diphenyl ether derivatives which show high herbicidal activity against weeds in upland fields and paddy fields while scarcely causing harm to crops. The present invention has been accomplished based on this finding.

The present invention is intended to provide a novel diphenyl ether derivative represented by the following formula (I):

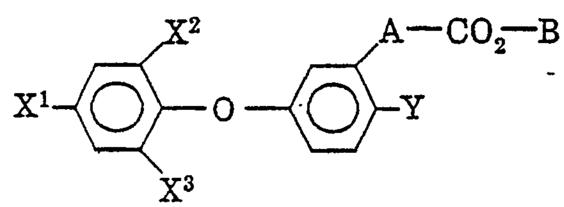

(I)

wherein $X^1$ represents a halogen atom or a $C_1$-$C_3$ haloalkyl group; $X^2$ and $X^3$ represent independently a hydrogen atom, a halogen atom, a trifluoromethyl group, a nitro group or a cyano group; Y represents a nitro group, a cyano group, a halogen atom, a $C_1$-$C_3$ haloalkyl group or a hydrogen atom; A represents a direct bond, a group represented by the formula:

$-CO_2CH(R^1)-$

wherein $R^1$ represents a hydrogen atom or a $C_1$-$C_3$ alkyl group, a group represented by the formula:

$-C(R^2) = NOCH(R^3)-$

wherein $R^2$ represents a hydrogen atom, a $C_1$-$C_4$ alkyl group which may be substituted with a $C_1$-$C_4$ alkoxy group, $C_1$-$C_4$ alkylthio group or halogen atom, or a $C_1$-$C_3$ alkoxy group, and $R^3$ represents a hydrogen atom or a $C_1$-$C_3$ alkyl group, or a group represented by the formula:

$-C(=NOR^4)OCH(R^3)-$

wherein $R^3$ is the same as defined above and $R^4$ represents a $C_1$-$C_3$ alkyl group; and B represents a group represented by the formula:

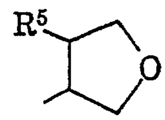

wherein $R^5$ represents a halogen atom; a hydroxyl group; an acyloxy group having up to 6 carbon atoms which may be substituted with a halogen atom, $C_1$-$C_6$ alkoxy group, $C_2$-$C_7$ alkoxycarbonyl group, aryl group which may be substituted with 1 to 3 halogen atoms and/or 1 to 3 $C_1$-$C_3$ alkyl groups or aryloxy group which may be substituted with 1 to 3 halogen atoms an/or 1 to 3 $C_1$-$C_3$ alkyl groups; a $C_4$-$C_9$ cycloalkylcarbonyloxy group; a benzoyloxy group which may be substituted with a halogen atom, $C_1$-$C_4$ alkyl group or $C_1$-$C_4$ alkoxy group; a $C_2$-$C_7$ alkoxycarbonyloxy group; a $C_1$-$C_4$ alkylsufonyloxy group; or a carbamoyloxy group which may be substituted with a $C_1$-$C_4$ alkyl group an/or $C_1$-$C_4$ alkoxy group.

It is also intended in this invention to provide a herbicidal composition containing the diphenyl ether derivative as active ingredient, and a process for producing the diphenyl ether derivative.

The diphenyl ether derivatives used as the active ingredient of the herbicidal composition in the present invention are represented by the above-described formula (I).

In the formula (I), $X^1$ represents a halogen atom or a $C_1$-$C_3$ haloalkyl group, preferably chlorine atom, bromine atom, a haloalkyl group such as trifluoromethyl group, trichloromethyl group, trifluoroethyl group, tetrafluoroethyl group and pentafluoroethyl group, and more preferably chlorine atom or trifluoromethyl group.

$X^2$ and $X^3$ represent independently a hydrogen atom, a halogen atom, a trifluoromethyl group, a nitro group or a cyano group, preferably hydrogen atom, fluorine atom, chlorine atom, bromine atom, trifluoromethyl group, nitro group or cyano group, and more preferably hydrogen atom or chlorine atom.

Y represents a nitro group, a cyano group, a halogen atom, a $C_1$-$C_3$ haloalkyl group or a hydrogen atom, preferably nitro group, cyano group, chlorine atom, bromine atom, iodine atom, a haloalkyl group such as trifluoromethyl group or hydrogen atom, and more preferably nitro group.

A represents a direct bond, a group represented by the formula:

$-CO_2CH(R^1)-$

a group represented by the formula:

$-C(R^2)=NOCH(R^3)-$

or a group represented by the formula:

$-C(=NOR^4)OCH(R^3)-$

In the above formulae, $R^1$ represents a hydrogen atom or a $C_1$-$C_3$ straight-chain or branched-chain alkyl group such as methyl group, ethyl group, n-propyl group and isopropyl group, preferably a hydrogen atom or methyl group, more preferably a methyl group. $R^2$ represents a hydrogen atom; a $C_1$-$C_4$ straight-chain or branched-chain alkyl group, such as methyl group, ethyl group, n-propyl group, isopropyl group, n-butyl group, isobutyl group, sec-butyl group and t-butyl group, which may be substituted with a $C_1$-$C_4$ straight-chain or branched-chain alkoxy group such as methoxy group, ethoxy group, n-propoxy group, isopropoxy group, n-butoxy group, isobutoxy group, sec-butoxy group and t-butoxy group, a $C_1$-$C_4$ straight-chain or branched-chain alkylthio group such as methylthio group, ethylthio group, n-propylthio group, isopropylthio group, n-butylthio group, isobutylthio group, sec-butylthio group and t-butylthio group or a halogen atom such as chlorine atom, bromine atom, iodine atom and fluorine atom; or a $C_1$-$C_3$ straight-chain or branched-chain alkoxy group. Preferred $R^2$ is a hydrogen atom, a $C_1$-$C_3$ straight-chain or branched-chain alkyl group which may substituted with a methoxy group, or a $C_1$-$C_3$ straight-chain or branched-chain alkoxy group, and more preferably a methyl group, ethyl group, methoxymethyl group, methoxy group or ethoxy group. $R^3$ represents a hydrogen atom or a $C_1$-$C_3$ straight-chain or branched-chain alkyl group such as methyl group, ethyl group, n-propyl group and isopropyl group, preferably a hydrogen atom, methyl group or ethyl group, and more preferably a hydrogen atom or methyl group. $R^4$ represents a $C_1$-$C_3$ straight-chain or branched-chain alkyl group such as methyl group, ethyl group, n-propyl group and isopropyl group, preferably a methyl group.

B represents a group represented by the formula:

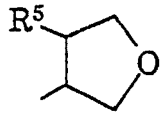

wherein $R^5$ represents a halogen atom such as chlorine atom, bromine atom, iodine atom and fluorine atom; a hydroxyl group; a saturated or unsaturated acyloxy group having up to 6 carbon atoms which may be substituted with one selected from the group consisting of a halogen atom such as chlorine atom, bromine atom, iodine atom and fluorine atom, a $C_1$-$C_6$ straight-chain or branched-chain alkoxy group such as methoxy group, ethoxy group, n-propoxy group, isopropoxy group, n-butoxy group, isobutoxy group, sec-butoxy group, t-butoxy group, n-pentyloxy group, isopentyloxy group, t-pentyloxy group and n-hexyloxy group, a $C_2$-$C_7$ straight-chain or branched-chain alkoxycarbonyl group such as methoxycarbonyl group, ethoxycarbonyl group, n-propoxycarbonyl group, isopropoxycarbonyl group, n-butoxycarbonyl group, isobutoxycarbonyl group, sec-butoxycarbonyl group, t-butoxycarbonyl group, n-pentyloxycarbonyl group and n-hexyloxycarbonyl group, an aryl group such as phenyl group and naphthyl group which may be substituted with 1 to 3 halogen atoms such as chlorine atom, bromine atom, iodine atom and fluorine atom and/or 1 to 3 $C_1$-$C_3$ straight-chain or branched-chain alkyl groups such as methyl group, ethyl group, n-propyl group and isopropyl group, and an aryloxy group such as phenoxy group and naphthyloxy group which may be substituted with 1 to 3 halogen atoms such as chlorine atom, bromine atom, iodine atom and fluorine atom and/or 1 to 3 $C_1$-$C_3$ straight-chain or branched-chain alkyl groups such as methyl group, ethyl group, n-propyl group and isopropyl group; a $C_4$-$C_9$ cycloalkylcarbonyloxy group such as cyclopropylcarbonyloxy group, cyclobutylcarbonyloxy group, cyclopentylcarbonyloxy group, cyclohexylcarbonyloxy group and cyclooctylcarbonyloxy group; a benzoyloxy group which may be substituted with a halogen atom such as chlorine atom, bromine atom, iodine atom and fluorine atom, a $C_1$-$C_4$ straight-chain or branched-chain alkyl group such as methyl group, ethyl group, n-propyl group, isopropyl group, n-butyl group, isobutyl group, sec-butyl group and t-butyl group, or a $C_1$-$C_4$ straight-chain or branched-chain alkoxy group such as methoxy group, ethoxy group, n-propoxy group, isopropoxy group, n-butoxy group, isobutoxy group, sec-butoxy group and t-butoxy group; a $C_2$-$C_7$ alkoxycarbonyloxy group such as methoxycarbonyloxy group, ethoxycarbonyloxy group, n-propoxycarbonyloxy group, isopropoxycarbonyloxy group, n-butoxycarbonyloxy group, isobutoxycarbonyloxy group, sec-butoxycarbonyloxy group, t-butoxycarbonyloxy group, n-pentyloxycarbonyloxy group and n-hexyloxycarbonyloxy group; a $C_1$-$C_4$ straight-chain or branched-chain alkylsulfonyloxy group such as methylsulfonyloxy group, ethylsulfonyloxy group, n-propylsulfonyloxy group, isopropylsulfonyloxy group, n-butylsulfonyloxy group, isobutylsulfonyloxy group, sec-butylsulfonyloxy group and t-butylsulfonyloxy group; a carbamoyloxy group which may be substituted with a $C_1$-$C_4$ straight-chain or branched-chain alkyl group such as methyl group, ethyl group, n-propyl group, isopropyl group, n-butyl group, isobutyl group, sec-butyl group and t-butyl group and/or a $C_1$-$C_4$ straight-chain or branched-chain alkoxy group such as methoxy group, ethoxy group, n-propoxy group, isopropoxy group, n-butoxy group, isobutoxy group, sec-butoxy group and t-butoxy group. Preferably, $R^5$ represents a chlorine atom; a bromine atom; a fluorine atom; a hydroxyl group; a saturated or unsaturated $C_2$-$C_4$ acyloxy group which may be substituted with one selected from the group consisting of chlorine atom, bromine atom, fluorine atom, a $C_1$-$C_3$ straight-chain or branched-chain alkoxy group, a $C_2$-$C_5$ straight-chain or branched-chain alkoxycarbonyl group, an aryl group which may be substituted with 1 to 3 halogen atoms and/or 1 to 3 $C_1$-$C_3$ straight-chain or branched-chain alkyl groups and an aryloxy group which may be substituted with 1 to 3 halogen atoms and/or 1 to 3 $C_1$-$C_3$ straight-chain or branched-chain alkyl groups; a $C_4$-$C_7$ cycloalkylcarbonyloxy group; a benzoyloxy group which may be substituted with a halogen atom; a $C_2$-$C_5$ straight-chain or branched-chain alkoxycarbonyloxy group; a $C_1$-$C_2$ alkylsulfonyloxy group; a carbamoyloxy group which may be substituted with a $C_1$-$C_2$ alkyl group and/or $C_1$-$C_2$ alkoxy group. More preferably, $R^5$ represents a chlorine atom; a bromine atom; a fluorine atom; a hydroxyl group; a $C_2$-$C_4$ acyloxy group which is saturated or has one double bond and which may be substituted with one selected from the group consisting of chlorine atom, fluorine atom, a $C_1$-$C_2$ alkoxy group, a $C_2$-$C_3$ alkoxycarbonyl group, a phenyl group which may be substituted with a chlorine atom and/or a methyl group; a $C_4$-$C_6$ cycloalkylcarbonyloxy group; a benzoyloxy group which may be substituted with a fluorine atom; a $C_2$-$C_3$ alkoxycarbonyloxy group; a $C_1$-$C_2$ alkylsulfonyloxy group; a carbamoyloxy group which may be substituted with a $C_1$-$C_2$ alkyl group and/or $C_1$-$C_2$ alkoxy group.

The compounds of the present invention represented by the formula (I) can be prepared, for example, according to the following reaction schemes (1) to (5).

(1)

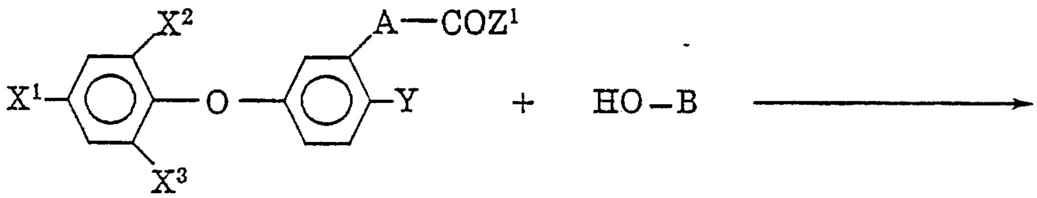

(II)

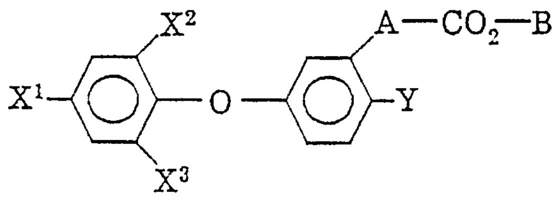

( I )

In the above reaction scheme, $Z^1$ represents a halogen atom such as chlorine atom, bromine atom, iodine atom and fluorine atom, and $X^1$, $X^2$, $X^3$, Y, A and B represents the same as defined above.

The above reaction is carried out in a solvent or without using any solvent in the presence or absence of a base. The solvents suitable for use in the above reaction are, for example, benzene, toluene, ethyl acetate, diethyl ether, diisopropyl ether, tetrahydrofuran, dioxane, N,N-dimethylformamide, N-methylpyrrolidone, 1,2- dimethoxyethane, methylene chloride, chloroform and the like. The bases usable in the above reaction include pyridine, picolines, quinoline, N,N-dimethyl(diethyl)aniline, triethylamine, sodium(potassium) hydrogencarbonate, sodium(potassium) carbonate, sodium(potassium) hydroxide and the like. The compound of the formula, HO-B, is used in an amount from 1 to 2 times equivalent to the compound of the formula (II). The base, when used, is used in an amount from 1 to 2 times equivalent to the compound of the formula (II). The reaction temperature is from -30 to 120 °C, preferably from -10 to 60 °C, and the reaction time is from 0.5 to 24 hours, preferably from 1 to 8 hours.

(2)

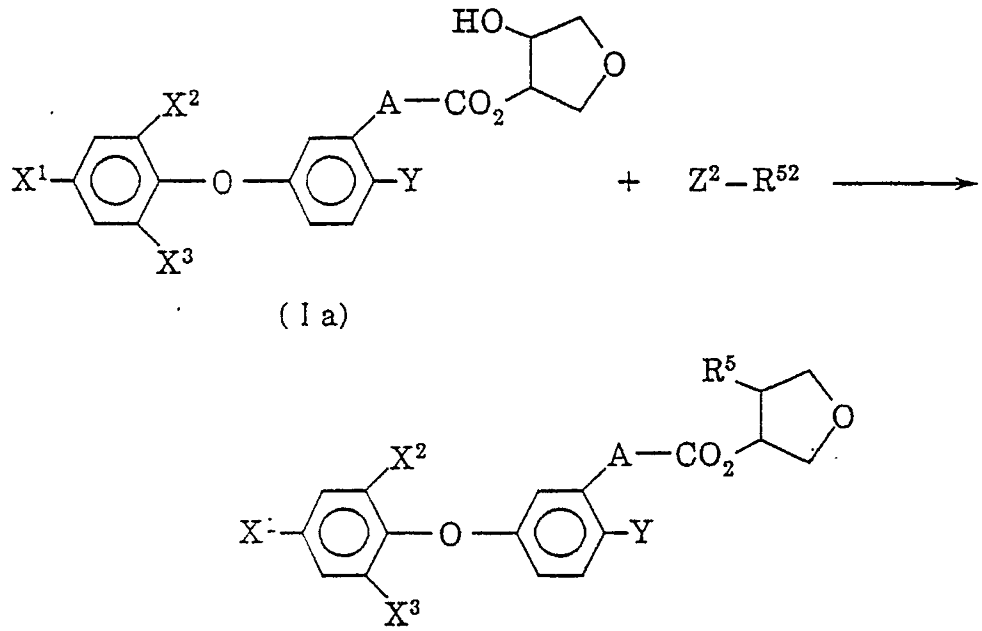

( I a)

( I b)

In the above reaction scheme, $R^5$ represents the same as defined above excepting hydroxyl group and halogen atom, $R^{52}$ represents a $C_2$-$C_6$ acyl group which may be substituted with one selected from the group consisting of a halogen atom, a $C_1$-$C_6$ alkoxy group, a $C_2$-$C_7$ alkoxycarbonyl group, an aryl group which may be substituted with 1 to 3 halogen atoms and/or 1 to 3 $C_1$-$C_3$ alkyl groups and an aryloxy group which may be substituted with 1 to 3 halogen atoms and/or 1 to 3 $C_1$-$C_3$ alkyl groups; a $C_4$-$C_9$ cycloalkylcarbonyl group; a benzoyl group which may be substituted with a halogen atom, $C_1$-$C_4$ alkyl group or $C_1$-$C_4$ alkoxy group; a $C_2$-$C_7$ alkoxycarbonyl group; a $C_1$-$C_4$ alkylsufonyl group; or a carbamoyl group which may be substituted with a $C_1$-$C_4$ alkyl group and/or $C_1$-$C_4$ alkoxy group, $Z^2$ represents a halogen atom such as chlorine atom and bromine atom, or a residue forming a symmetric or unsymmetric acid anhydride with $R^{52}$ when $R^{52}$ is an acyl group specified above, and $X^1$, $X^2$, $X^3$, Y and A represents the same as defined above.

This reaction is performed in a solvent or without any solvent in the presence or absence of a base. When using a solvent in the above reaction, suitable solvents are, for example, benzene, toluene, ethyl acetate, diethyl ether, diisopropyl ether, tetrahydrofuran, dioxane, N,N-dimethylformamide, N-methylpyrrolidone, 1,2-dimethoxyethane, methylene chloride, chloroform and the like. The bases usable in the above reaction include pyridine, picolines, quinoline, N,N-dimethyl(diethyl)aniline, triethylamine, sodium(potassium) hydrogencarbonate, sodium(potassium) carbonate, sodium(potassium) hydroxide, sodium hydride and the like. The amount used of the compound of the formula, $Z^2$-$R^{52}$, is from 1 to 2 times equivalent to the compound of the formula (Ia). The base may be used in an amount from 1 to 2 times equivalent to the compound of the formula (Ia). The reaction temperature is from -30 to 120 °C, preferably from -10C to 60 °C. The reaction time is from 0.5 to 24 hours, preferably 1 to 8 hours.

(3)

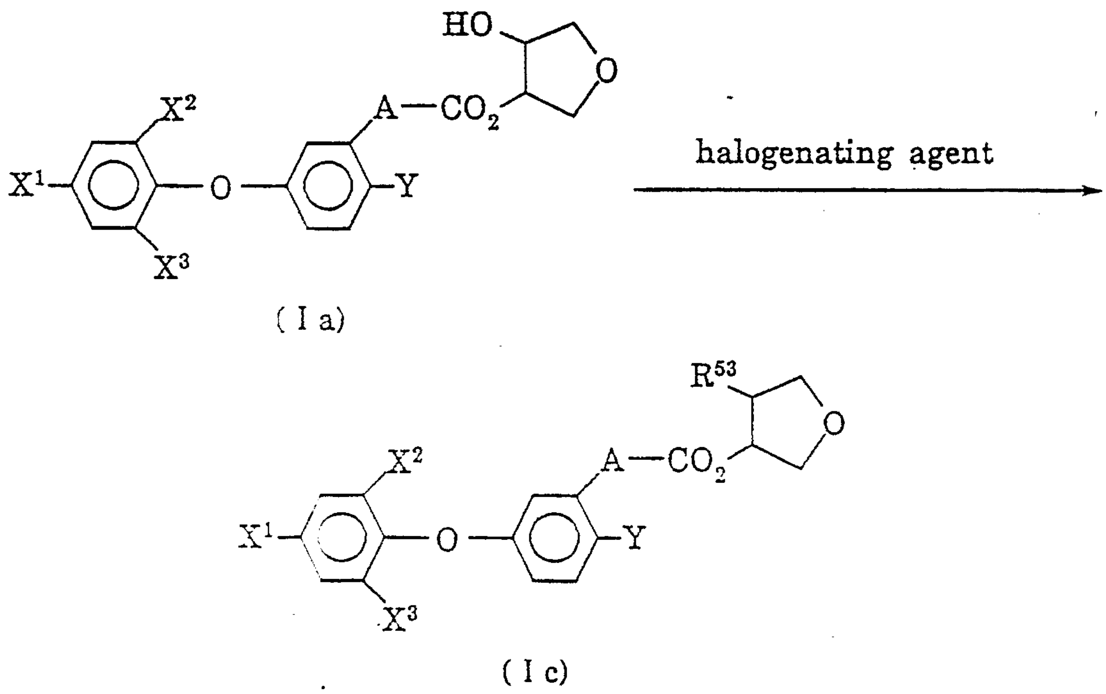


In the above reaction scheme, $R^{53}$ represents a halogen atom, and $X^1$, $X^2$, $X^3$, Y and A represent the same as defined above.

The above reaction is conducted in a solvent or without using any solvent in the presence or absence of a base. The halogenating agent used in the above reaction is, for example, hydrogen fluoride, hydrogen fluoride complex such as hydrogen fluoride-pyridine complex and hydrogen fluoride-melamine complex, 2-chloro-1,1,2- trifluorotriethylamine, diethyl-1,1,2,3,3,3-hexafluoropropylamine, diethylaminosullur trifluoride, phenylfluoro phosphoranes, thionyl chloride, phosphorus oxychloride, hydrogen chloride, thionyl bromide, phosphorus tribromide, hydrogen bromide, phosphorus triiodide, hydrogen iodide and the like. The halogenating agent is used in an amount from 1 to 30 times equivalent to the compound of the formula (Ia). The solvents suitable for use in the above reaction are, for example, benzene, toluene, diethyl ether, 1,2-dimethoxyethane, tetrahydrofuran, dioxane, digrime, acetonitrile, methylene chloride, chloroform, carbon tetrachloride, ethyl acetate, N,N-dimethylformamide, N-methylpyrrolidone, acetic acid and water. These solvents may be used in admixture. The bases that can be favorably used in the above reaction include pyridine, picolines, lutidines, quinoline, tri(lower alkyl)amines, diisopropylamine, dicyclohexylamine, and the like. The base may be used in an amount from 1 to 2 times equivalent to the compound of the formula (Ia). The reaction can be conducted in a temperature from -30 to 200 °C, preferably from -10 to 150 °C, and for a period from 0.1 to 30 hours, preferably from 0.1 to 12 hours.

(4)

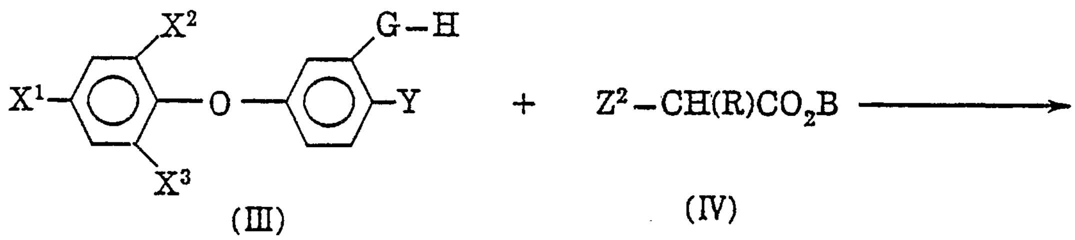

(III) (IV)

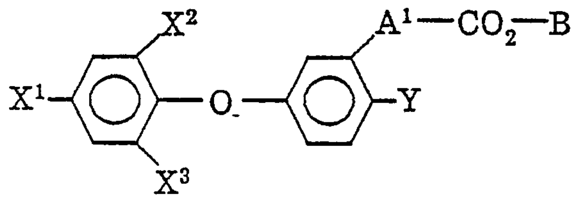

( I d)

In the above reaction scheme, G represents a group represented by the formula $-CO_2-$, $-C(R^{22})=NO-$ or $-CONH(OR^4)-$, $R^{22}$ represents a hydrogen atom or a $C_1$-$C_4$ alkyl group which may be substituted with a $C_1$-$C_4$ alkoxy group, $C_1$-$C_4$ alkylthio group or halogen atom, R is equal to $R^1$ when G represents $-CO_2-$ and equal to $R^3$ when G represents $-C(R^{22})=NO-$ or $-CONH(OR^4)-$, $A^1$ represents a group of the formula $-CO_2CH(R^1)-$ when G is $-CO_2-$and represents a group of the formula $-C(R^{22}) = NOCH(R^3)-$ or $-C(=NOR^4)-OCH(R^3)-$ when G is a group of the formula $-C(R^{22})=NO-$or $-CONH(OR^4)-$, and $X^1$, $X^2$, $X^3$, Y, B, $R^1$, $R^3$, $R^4$ and $Z^2$ are the same as defined above.

This reaction is carried out in a solvent or without solvent in the presence of a base. When using a solvent in the above reaction, the suitable solvents are, for example, methanol, ethanol, 2-propanol, acetone, ethyl methyl ketone, diethyl ether, diisopropyl ether, ethyl acetate, benzene, toluene, xylene, tetrahydrofuran, dioxane, 1,2-dimethoxyethane, acetonitrile, N,N-dimethylformamide, n-methylpyrrolidone and the like. These solvents may be used in admixture. The bases used in the above reaction are, for example, sodium(potassium) hydrogencarbonate, sodium(potassium) carbonate, sodium(potassium, barium or calcium) hydroxide, pyridine, picolines, quinoline, N,N-dimethyl(diethyl)aniline, triethylamine, sodlum-(potassium) lower alkoxide, sodium(potassium) hydride, metallic sodium, potassium fluoride and the like. The base is used in an amount from 1 to 2 times equivalent to the compound of the formula (III). The amount of the compound of (IV) is from 1 to 2 times'equivalent to the compound of the formula (III). The reaction is carried out at a temperature from -30 to 200 °C, preferably from -10 to 150 °C, for a period from 0.5 to 48 hours, preferably from 1 to 18 hours.

(5)

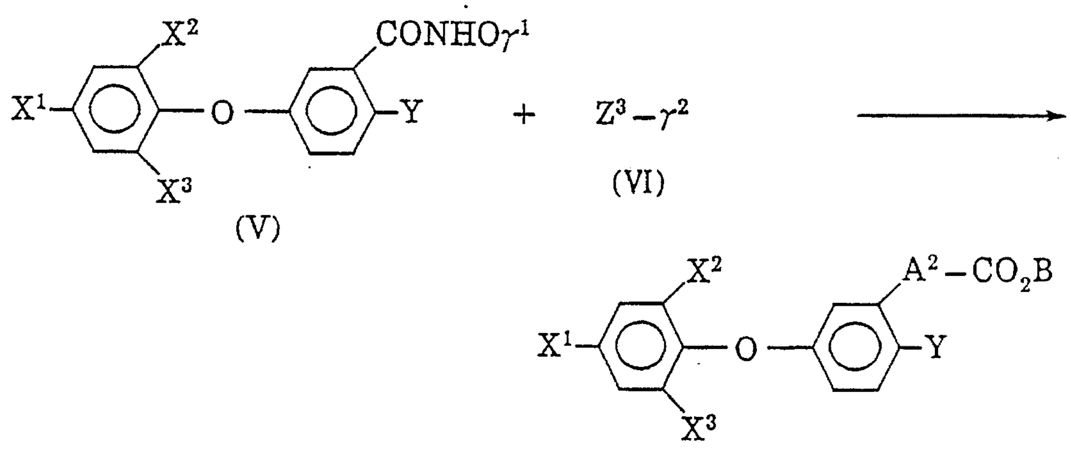

(V) (VI) (I e)

In the above reaction formula, $\gamma^1$ represents $-CH(R^3)CO_2B$ or $R^4$, $\gamma^2$ represents $R^{23}$ when $\gamma^1$ is $-CH(R^3)-CO_2B$ or represents $-CH(R^3)CO_2B$ when $\gamma^1$ is $R^4$, $Z^3$ represents a halogen atom or, a phenylsulfonyloxy or alkylsulfonyloxy group which may be substituted, such as p-toluenesulfonyloxy group, methanesulfonyloxy group and trifluoromethanesulfonyloxy group, $A^2$ represents $-C(R^{23})=NOCH(R^3)-$or $-C(=NOR^4)OCH(R^3)-$, $R^{23}$ represents a $C_1$-$C_4$ alkyl group which may be substituted with a $C_1$-$C_4$ alkoxy group, $C_1$-$C_4$ alkylthio group or halogen atom, and $X^1$, $X^2$, $X^3$, Y, $R^3$, $R^4$ and B are the same as defined above.

This reaction is carried out in a solvent or without solvent in the presence of a base. When using a solvent in the above reaction, the suitable solvents are, for example, methanol, ethanol, 2-propanol, acetone, ethyl methyl ketone, diethyl ether, diisopropyl ether, ethyl acetate, benzene, toluene, xylene, tetrahydrofuran, dioxane, 1,2-dimethoxyethane, acetonitrile, N,N-dimethylformamide, N-methylpyrrolidone and the like. These solvents may be used in admixture. The bases used in the above reaction are, for example, sodium(potassium) hydrogencarbonate, sodium(potassium) carbonate, sodium(potassium, barium or calcium) hydroxide, pyridine, picolines, quinoline, N,N-dimethyl(diethyl)aniline, triethylamine, sodium-(potassium) lower alkoxide, sodium(potassium) hydride, metallic sodium, potassium fluoride and the like. The base is used in an amount from 1 to 2 times equivalent to the compound of the formula (V). The amount of the compound of (VI) is from 1 to 2 times equivalent to the compound of the formula (V). The reaction is carried out at a temperature from -30 to 200 °C, preferably from -10 to 150 °C, for a period from 0.5 to 48 hours, preferably from 1 to 18 hours.

The compounds of this invention obtained in the manner described above include isomers such as optical isomers, diastereomers, etc. The compound of the present invention includes all of these isomers. These isomers, either singly or in any combination, can be used as an active ingredient of the herbicidal composition.

The compounds of this invention may be used as herbicide either directly or in the form of a composition such as wettable powder, granules, emulsion, flowable agent, etc., which can be prepared according to the conventional methods by using a carrier, surfactant or other suitable adjuvant. As for such carrier and surfactant, there can be used, for instance, those described in Japanese Patent Application Laid-Open(KOKAI) No. 60-25986(1985). Also, the herbicidal composition containing the compound of this invention can be used in combination with other agricultural chemicals such as insecticide, fungicide, herbicide, growth regulator and fertilizer.

The amount of the herbicidal composition of this invention to be applied in practical use is variable depending on various factors such as type of compound used, species of weed to be treated, application time, method of application and quality of soil, but the application amount is usually from 0.005 to 2 kg, preferably from 0.01 to 1 kg per hectare as the amount of the active ingredient.

The diphenyl ether derivative of the present invention shows an extremely high herbicidal activity against harmful weeds in upland fields and paddy fields in either pre-emergence treatment or post-emergence treatment. The weed in upland field which can be controlled by the compound of the present invention are, for instance, Chenopodium album L., Chenopodium album L. var. centrorubrum, Persicaria blumei gross, Polygonum persicaria L., Amaranthus blitum L., Amaranthus retroflexus, Stellaria media, Laminum amplexicaule, Digitaria adscendens, Eleusine indica Gaertner, Panicum crus-galli L., Setaria

viridis, Alopecurus aequalis, and Cyperus microiria, and the weed in paddy fields are, for instance, Rotala indica, Lindernia pyxidaria L., Monochoria vaginalis, Dopatrium junceum Hamilton, Elatine triandra Schk, Alisma canaliculatum, Echinochloa crus-galli, var. hispidula and Cyperus difformis. The compound of this invention scarcely shows phytotoxicity against crops such as soybean, cotton, sunflower, potato, rice, wheat, barley, corn, sugar cane, etc., in either pre-emergnece treatment or treatment during growing up, and thus, usable as a selective herbicide.

Furthermore, the compound of this invention shows a high herbicidal activity against weeds, which have hitherto been considered difficult to be conzoled, such as Abutilon avicennae, Ipomoea purpurea, Datura stramonium L. var. chalybea Koch, Brassica kaber, Galium aparine, Viola tricolor, Matricaria chamomilla, etc., in upland fields, and Sagittaria pygmaea, Sagittaria trifolia L., Scirpus juncoides, Cyperus serotinus etc., in paddy fields.

In use of the compounds of this invention as a herbicidal component, there can be noted a slight degree of variation in herbicidal activity according to the type of modified functional group or the position thereof, or a slightly low herbicidal activity when applied to grown weeds. In these cases, it is possible to make sure or enhance the herbicidal activity of the herbicidal composition by a combined use of other herbicides. The herbicides that can be combinedly used with the compounds of this invention are those exemplified below.

Pyrazole type herbicides: 4-(2,4-dichlorobenzoyl)-1,3-dimethylpyrazol-5-yl p-toluenesulfonate, 4-(2,4-dichlorobenzoyl)-1,3-dimethyl-5-phenacyloxypyrazole, 4-(2,4-dichloro-3-methylbenzoyl)-1,3-dimethyl-5-(4-methylphenacyloxy)pyrazole, 4-(2,4-dichlorobenzoyl-1-methyl-5-phenacyloxypyrazole, etc.;

Sulfonylurea type herbicides: methyl 2-(4,6-dimethoxypyrimidin-2-ylcarbamoylaminosulfonylmethyl)-benzoate, ethyl 5-(4,6-dimethoxypyrimidin-2-yl-carbamoylaminosulfonyl)-1-methylpyrazole-4-carboxylate 2-chloro-N-(4-methoxy-6-methyl-1,3,5-triazin-2-ylaminocarbonyl) benzenesulfonemide, methyl 2-(4-methoxy-6-methyl-1,3,5-triazin-2-ylcarbamoylaminosulfonyl)-benzoate, methyl 2-(4,6-dimethylpyrimidin-2-ylcarbamoylaminosulfonyl)benzoate, ethyl 2-(4-chloro-6- methoxypyrimidin-2-ylcarbamoylaminosulfonyl)-benzoate, etc.;

Phenoxy type herbicides: 2,4-dichlorophenoxyacetic acid and derivatives thereof, 4-chloro-2-methylphenoxyacetic acid and derivatives thereof, 4-(4-chloro-2-methylphenoxy)butyric acid and derivatives thereof, S-ethyl 4-chloro-2-methylphenoxythioacetate, 2-(2-naphthoxy)propionanilide, 2-(2,4-dichloro-3-methylphenoxy)propionanilide, butyl 2-[4-(5-trifluoromethyl-2-pyridyloxy)phenoxy]propionate, etc.;

Haloacetanilide type herbicides: 2-chloro-2′,6′-diethyl-N-methoxymethylacetanilide, 2-chloro-2′,6′-diethyl-N-butoxymethylacetanilide, 2-chloro-2′-ethyl-6′-methyl-N-(2-methoxy-1-methylethyl)acetanilide, 2-chloro-2′,6′-diethyl-N-propoxyethylacetanilide, ethyl N-chloroacetyl-N-(2,6-diethylphenyl)aminoacetate, 2-chloro-2′,6′-dimethyl-N-(3-methoxy-2-tenylmethyl)acetanilide, etc.;

Acid amide type herbicides: 3′,4′-dichloro-propionanilide, 2′,3′-dichloro-4-ethoxymethoxybenzanilide, 2-bromo-3,3-dimethyl-N-(α,α-dimethylbenzyl)butyramide, 2-benzothiazol-2-yloxy-N-methylacetanilide, 2′,4′-difluoro-2-(3-trifluoromethyl- phenoxy)nicotinanilide, 2,6-dimethoxy-N-[3-(1-ethyl-1-methylpropyl) isoxazol-5-yl]benzamide, etc.;

Carbamate type herbicides: S-(4-chlorobenzyl)-N,N-diethylthiolcarbamate, S-ethyl-N,N-hexamethylenethiolcarbamate, N,N-hexamethylene-S-isopropylthiolcarbamate, S-benzyl-N-ethyl-N-(1,2-dimethylpropyl)thiolcarbamate, S-(1-methyl-1-phenethyl) pyperidin-1-carbothioate, O-(3-t-butylphenyl)-N-(6-methoxypyridin-2-yl)-N-methylthiocarbamate, S-ethyl-N,N-di-n-propylthiolcarbamate, S-ethyl-N,N-diisobutylthiolcarbamate, isopropyl-N-(3-chlorophenyl)carbamate, 3-methoxvcarbonylaminophenyl-N-(3-methylphenyl)-carbamate, S-(2,3-dichloroallyl)-N,N-diisopropylthiolcarbamate, S-(2,3,3-trichloroallyl)-N,N-diisopropylthiolcarbamate, methyl-N-(4-aminobenzenesulfonyl)carbamate, etc.;

Urea type herbicides: 1-(α,α-dimethylbenzyl)-3-(4-methylphenyl)urea, 3-(benzothiazol-2-yl)-1,3-dimethylurea, 3-(3,4-dichlorophenyl)-1-methoxy-1-methylurea, 3-(3,4-dichlorophenyl)-1,1-dimethylurea, 3-[4-(4-methylphenethyloxy)phenyl]-1-methoxy-1-methylurea, 3-(4-isopropylphenyl)-1,1-dimethylurea, 1-(2-substituted benzyl)-3-(α,α-dimethylbenzyl)urea, etc.,

Diphenyl ether type herbidices: 2,4,6-trichloro-4′-nitrodiphenyl ether, 2,4-dichloro-3′-methoxy-4′-nitrodiphenyl ether, methy 5-(2,4-dichlorophenoxy)-2-nitrobenzoate, 3-[5-(2-chloro-4-trifluoromethylphenoxy)-2-nitrophenoxy]tetrahydrofuran, 5-(2-chloro-4-trifluoromethylphenoxy)-2-nitrobenzoic acid and a salt thereof, 2-chloro-3′-ethoxy-4′-nitro-4-trifluoro-methyldiphenyl ether, 1-ethoxycarbonylethyl 5-(2-chloro-4-trifluoromethylphenoxy)-2-nitrobenzoate, 5-(2-chloro-4-triflouromethylphenoxy)-N-methanesulfonyl-2-nitrobenzamide, methyl 5-(2-chloro-4-trifluoromethylphenoxy)-2-nitroacetophenoneoxime-O-acetate, 3-amino-2-chloro-4-nitrodiphenyl ether, etc.;

Triazine type herbicides: 2-chloro-4-ethylamino-6-isopropylamino-1,3,5-triazine, 2,4-bis(ethylamino)-6-methylthic-1,3,5-triazine, 2-ethylamino-4-(1,2-dimethylpropylamino)-6-methylthio-1,3,5-triazine, 2,4-bis-

(isopropylamino)-6-methylthio-1,3,5-triazine, 4-amino-6-t-butyl-3-methylthio-1,2,4-triazin-5(4H)-one, 4-amino-6-t-butyl-3-ethylthio-1,2,3-triazin-5(4H)-one, 2-chloro-4,6-bis(ethylamino)-1,3,5-triazine, 2-(2-chloro-4-ethylamine-1,3,5-triazin-6-ylamino)-2-methylpropinitrile, etc.;

Dinitroaniline type herbicides: 2,6-dinitro-N,N-dipropyl-4-trifluoromethylaniline, N-(1-ethylpropyl)-3,4-dimethyl-2,6-dinitroaniline, 3,5-dinitro-N,N-dipropylsulfanilamide, etc.;

Nitrile type herbicides: 4-hydroxy-3,5-diiodobenzonitrile, 3,5-dibromo-4-hydroxybenzonitrile 2,6-dichlorobenzonitrile, etc.;

Phosphorus containing herbicides: O-ethyl -O- (5-methyl-2-nitrophenyl)-N-sec-butylphosphoramidate, S-(2-benzenesulfonylaminoethyl) -O, O-diisopropylphosphorodithioate, S-(2-methylpiperidin-1-yl) carbonylmethyl-O,O-dipropylphosphorodithioate, N-(phosphonomethyl)glycine, ammonium (3-amino-3-carboxy)propylmethylphosphinate, sodium (2-amino-4-methylphosphino)butyrylalanylalaninate, etc.;

Quaternary ammonium salt type herbicides: 1, 1′-ethylene-2,2′-bipyridylium dibromide, 1,1′-dimethyl-4,4′-bipyridylium dichloride, etc.;

Other herbicides: 3,6-dichloro-2-methoxybenzoic acid, 3,7-dichloroquinoline-8-carboxylic acid, pentachlorophenol, 2-sec-butyl-4,5-dinitrophenol, 2-amino-3-chloro-1,4-napthoquinone, 1,2-dihydropyridazin-3,6-dione, 3-(2-methylphenoxy)pyridazine, 3-isopropyl-1H-2,1,3-benzothiadiazin-4(3H)-one-2,2-dioxide, 2,2-dichloropropionic acid, 2,2,3,3-tetrafluoropropionic acid, methyl 6-(4-isopropyl-4-methyl-5-oxo-2-imidazolin-2-yl)-3(4)-methylbenzoate, 2-(4-isopropyl-4-methyl-5-oxo-2-imidazolin-2-yl)nicotinic acid and a salt thereof, 2-(4-isopropyl-4-methyl-5-oxo-2-imidazolin-2-yl)quinoline-3- carboxylic acid, 5-ethyl-2-(4-isopropyl-4-methyl-5-oxo-2-imidazolin-2-yl)nicotinic acid, 1-methyl-4-(1-methylethyl)-2-(2-methylphenylmethoxy)-7-oxabicyclo-[2,2,1]heptane, 1-(3-methylphenyl)-5-phenyl-1H-1,2,4-triazol-3-carboximide, 2-N-ethoxybutylimidoyl)-5-(2-ethylthiopropyl)-3-hydoxy-2-cyclohexen-1-one, 2-(3,5-dichlorophenyl)-2-(2,2,2-trichloroethyl)oxirane, N-[4-(4-chlorobenzyloxy)phenyl]-3,4,5,6-tetrahydrophthalimideN-(4-chloro-2-fluoro-5-propargyloxyphenyl)-3,4,5,6-tetrahydrophthalimide, 3-(2,4-dichloro-5-isopropoxy-phenyl)-5-t-butyl-1,3,4-oxdiazol-2(3H)-one, 4-methoxy-3,3′-dimethylbenzophenone, 2-ethoxy-2,3-dihydro-3,3-dimethyl-5-benzofuranylemethanesulfonate, 1-methyl-3-phenyl-5-(3-trifluoromethylphenyl)-pyridin-4(1H)-one, 2-(2-chlorophenyl)methyl-4,4-dimethyl-3-isoxazolidinone, (E), (E)-2-[1-(3-chloropropen-2-yloxyimino)butyl]-5-(2-ethylthiopropyl)-3-hydroxy-2-cyclohexen-1-one, 2-(1-ethoxyiminopropyl)-3-hydroxy-5-mesityl-2-cyclohexen-1-one, etc.

These may be used in combination of two or more.

The present invention will be described in further detail hereinbelow, but it is to be understood that these examples are intended to be merely illustrative and not to be construed as limiting the scope of the invention.

## Example 1:

Preparation of 3-[5-(2-chloro-4-trifluoromethylphenoxy)-2-nitrobenzoyloxy]-4-hydroxytetrahydrofuran

A mixture of 3.53 g of 5-(-2-chloro-4-trifluoromethylphenoxy)-2-nitrobenzoic acid and thionyl chloride was heated to reflux for 2 hours.

Then, excess thionyl chloride was distilled off and the residue was dissolved in 5 ml of dry ethyl acetate. Into a mixture of 1.52 g of 3,4-dihydroxytetrahydrofuran, 1.97 g of triethylamine and 30 ml of ethyl acetate cooled to 0 °C, the thus obtained ethyl acetate solution of the acid chloride was added dropwise with stirring. Alter stirring for 3 hours at room temperature, the reaction mixture was washed with water followed by brine, and the separated organic layer was dried over anhydrous sodium sulfate. After removing the solvent by evaporation, the residue was purified by silica gel column chromatography with ethyl acetate/n-hexane (1 : 1) to give 2.20 g of compound No. 2 described in Table 1.

Melting point : 92 to 94 °C

IR spectrum (KBr) $cm^{-1}$ : 3400, 1735, 1575, 1525, 1315, 1260, 1130, 1075

$^1$H-NMR spectrum ($CDC1_3$-TMS) $\delta$ : 2.14 (1H, d), 3.63-4.26 (4H, m), 4.60 (1H, quint), 5.43 (1H, q), 7.04-8.14-(6H, m)

## Example 2:

Preparation of 3-[5-(2-chloro-4-trifluoromethylphenoxy)-2-nitrobenzoyloxy]-4-methoxyacetoxytetrahydrofuran

A mixture of 2.89 g of 5-(2-chloro-4-trifluoromethylphenoxy)-2-nitrobenzoic acid and 2 ml of thionyl chloride was heated to reflux for 2 hours. After distilling off the excess thionyl chloride, the residue was dissolved in 4 ml of benzene. Into a mixture of 1.54 g of 3-hydroxy-4-methoxyacetoxytetrahydrofuran, 1.32 g of triethylamine and 25 ml of benzene, the thus obtained benzene solution of the acid chloride was added dropwise wlth stirring at room temperature. Alter stirring for 3 hours, the reaction mixture was washed with water followed by brine, and the separated organic layer was dried over anhydrous sodium sulfate. Alter evaporation, the residue was purified by silica gel column chromatography with ethyl acetate/n-hexane (2 : 3) to give 2.60 g of compound No. 57 described in Table 1. Melting point : 72 - 72.5 °C
IR spectrum (KBr) $cm^{-1}$: 1760, 1735, 1580, 1530, 1325, 1280, 1130, 1080
$^1$H-NMR spectrum ($CC1_4$-TMS) $\delta$ : 3.32 (3H, s), 3.91 (2H, s), 3.75, 4.12 (each 2H, m), 3.57, 5.54 (each 1H, q), 7.02-8.07 (6H, m)

Example 3:

Preparation of 3-acetoxy-4-[5-(2-chloro-4-trifluoromethylphenoxy)-2-nitrobenzoyloxy]tetrahydrofuran

To a mixture of 0.68 g of compound No. 2 of Table 1 obtained in Example 1, 0.20 g of triethylamine and 10 ml of dry ethyl acetate, was added 0.14 g of acetyl chloride dropwise with stirring under water cooling. After stirring for 5 hours at room temperature, the reaction mixture was washed with water followed by brine, and the separated organic layer was dried over anhydrous sodium sulfate. Then the solvent was removed by evaporation and the residue was purified by silica gel column chromatography with ethyl acetate/n-hexane (1 : 2) to give 0.60 g of compound No. 26 shown in Table 1.
Melting point : 88 to 89 °C
IR spectrum (KBr) $cm^{-1}$: 1740, 1575, 1525, 1320, 1290, 1235, 1125, 1075
$^1$H-NMR spectrum ($CC1_4$-TMS) $\delta$ : 1.98 (3H, s), 3.60-4.22 (4H, m), 5.28, 5.48 (each 1H, q), 7.02-8.05 (6H, m)

Example 4:

Preparation of 3-[5-(2-chloro-4-trifluoromethylphenoxy)-2-nitro benzoyloxy]-4-(N-methoxy-N-methylcarbamoyloxy)tetrahydrofuran

After dissolving 0.80 g of compound No. 2 of Table 1 obtained in Example 1 in 4 ml of pyridine, to this was added 0.27 g of N- methoxy-N-methylcarbamoyl chloride. The solution was allowed to stand overnight at room temperature and quenched with water. The resulting mixture was neutralized with concentrated hydrochloric acid and then extracted with three portions of ether. The organic layer was washed with brine and dried over anhydrous sodium sulfate. Then the solvent was removed by evaporation and the residue was purified by silica gel column chromatography with ethyl acetete(n-hexane (2 : 3) to give 0.58 g of compound No. 82 described in Table 1. Amorphous solid
IR spectrum (Cap.) $cm^{-1}$ : 1740, 1720, 1580, 1530, 1220, 1280, 1130, 1080
$^1$H-NMR spectrum ($CC1_4$-TMS) $\delta$ : 3.00 (3H, s), 3.50 (3H, s), 3.70-4.21 (4H, m), 5.26, 5.53 (each 1H, q), 6.97-8.08 (6H, m)

Example 5:

Preparation of 3-[5-(2-chloro-4-trifluoromethylphenoxy)-2-nitrobenzoyloxy]-4-fluorotetrahydrofuran

A mixture of 1.34 g of compound No. 2 of Table 1 obtained in Example 1, 1.35 ml of N,N-diethylaminosulfur trifluoride and 15 ml of 1,2-dimethoxyethane was stirred at 70 °C for 5 hours. This mixture was cooled to room temperature and further to below 0 °C. Then 1.5 ml of ethylene glycol was added and the mixture was stirred for 0.5 hour. To the resulting reaction mixture, was added a saturated sodium hydrogencarbonate solution, and take solution was extracted with three portions of ether. The

organic layer was washed with water followed by brine, dried over anhydrous sodium sulfate and concentrated under reduced pressure. The residue was purified by a silica gel column chromatography with ethyl acetate/n-hexane (1 : 4) to give 1.00 g of compound No. 12 described in Table 1.
$n_D^{25}$ : 1.5430
IR spectrum (Cap.) $cm^{-1}$ : 1745, 1580, 1530, 1320, 1285, 1130, 1080 $^1$H-NMR spectrum ($CCl_4$TMS) δ : 3.69-4.24 (4H, m), 5.13 (1H, dm), 5.47 (1H, m), 6.96-8.05 (6H, m)

Example 6:

Preparation of 3-chloro-4-[5-(2-chloro-4-trifluoromethylphenoxy)-2-nitrobenzoyloxy]tetrahydrofuran

To a mixture of 0.67 g of compound No. 2 described in Table 1 and obtained in Example 1, 0.26 g of pyridine and 10 ml of methylene chloride, was added 0.38 g of thionyl chloride with stirring at room temperature. Alter stirring for 5 hours at the same temperature, the mixture was allowed to stand overnight. Then the reaction mixture was concentrated under reduced pressure, and the residue was taken up in ether and washed with 2N hydrochloric acid. water followed by brine. Alter drying over anhydrous sodium sulfate, the solvent was removed and the residue was purified by silica gel column chromatography with ethyl acetate/n-hexane (1 : 4) to give 0.20 g of compound No. 20 of Table 1 as amorphous solid.
IR spectrum (Cap.) $cm^{-1}$ : 1740, 1580, 1525, 1280, 1125, 1075 $^1$H-NMR spectrum ($CCl_4$-TMS) δ : 3.84-4.33 (4H, m), 4.43, 5.47 (each 1H, m), 7.01-8.11 (6H, m)

Example 7:

Preparation of 3-acetoxy-4-[2-(5-(2-chloro-4-trifluoromethylphenoxy)-2-nitrobenzoyloxy)propionyloxy]-tetrahydrofuran

After dissolving 1.81 g of 5-(2-chloro-4-trifluoromethylphenoxy)-2-nitrobenzoic acid in 10 ml of N,N-dimethylformamide, to this was added 0.24 g of 60% oil sodium hydride with stirring at room temperature. After foaming ceased, was added 1.41 g of 3-acetoxy-4(2-bromopropionyloxy)tetrahydrofuran and the mixture was stirred at 100 °C for 8 hours. Alter cooling to room temperature, the mixture was extracted with five portions of ether and the combined organic layer was washed with brine and dried over anhydrous sodium sulfate.

Then the solvent was removed by evaporation and the residue was purified by a silica gel column chromatography with ethyl acetate/n-hexane (1 : 2) to give 0.70 g of compound No. 33 of Table 1 as a viscous liquid.
IR spectrum (Cap.) $cm^{-1}$: 1740, 1580, 1530, 1320, 1235, 1125, 1075
$^1$H-NMR spectrum ($CC1_4$-TMS) δ : 1.58 (3H, s), 1.98 (3H, s), 3.68, 3.98 (each 2H, m), 5.30 (3H, m), 7.00-8.11 (6H, m)

Example 8:

Preparation of 3-acetoxy-4-[1-(5-(2-chloro-4-trifluoromethylphenoxy)-2-nitrophenyl)-ethylideneiminoxyacetoxy]tetrahydrofuran

(a) Preparation of 5-(2-chloro-4-trifluoromethylphenoxy)-2-nitroacetophenone oxime (mixture of isomers) To a mixture of 5.20 g of 5-(-2-chloro-4-trifluoromethylphenoxy)-2-nitroacetophenone, 25 ml of ethanol and 25 ml of benzene, was added a mixture of 3.01 g of hydroxylamine hydrochloride, 4.48 g of triethylamine and 30 ml of ethanol, and the mixture was refluxed with stirring for 8 hours. After distilled off the solvent, the residue was diluted with water and extracted with three portions of ether. The organic layer was washed with brine (two times) and dried over anhydrous sodium sulfate. Then the solvent was removed and the residue was triturated with n-hexane. Collecting by suction, the titled compound (Compound P1) was

obtained quantitatively.

(b) Separation of the isomers

To a mixture of 9.12 g of 5-(2-chloro-4-trifluoromethyl-phenoxy)-2-nitroacetophenone, 50 ml of ethanol and 40 ml of benzene, was added a mixture of 5.26 g of hydroxylamine hydrochloride, 7.76 g of triethylamine and 50 ml of ethanol, and the mixture was reacted in the same manner as in (a). Thin-layer silica gel chromatography (with ethyl acetate/n-hexane (1 : 4) ) of the reaction mixture showed disappearance of the starting substances and formation of two substances at $R_f$ 0.35 and $R_f$ 0.28. After evaporation, the residue was diluted with water and extracted with three portions of ether, and the organic layer was washed with brine and dried over anhydrous sodium sulfate. After removing the solvent, the residue was subjected to separation by silica gel column chromatography with ethyl acetate/n-hexane (1 : 3) to give 4.72 g of a higher $R_f$ substance (compound P2), 1.62 g of a lower $R_f$ substance (compound P3) and 3.10 g of a mixture of those substances. The compounds P2 and P3 are the isomers with respect to the oxime linkage.

Compound P2

Melting point : 130 to 130.5 °C

IR spectrum (KBr) $cm^{-1}$ : 3300, 1575, 1520, 1340, 1320, 1260, 1235, 1165, 1120, 1075

$^1$H-NMR spectrum ($CDCl_3$-TMS) $\delta$ : 2.17 (3H, s, $CH_3$), 8.24 (1H, s, OH)

Compound P3

Melting point : 162.5 to 163 °C

IR spectrum (KBr) $cm^{-1}$ : 3280, 1580, 1520, 1320, 1235, 1125, 1080

$^1$H-NMR spectrum ($CDCl_3$-TMS) $\delta$ : 2.22 (3H, s, $CH_3$), 7.72 (1H, s, OH)

(c) Preparation of 3-acetoxy-4-[1-(5-(2-chloro-4-trifluoromethylphenoxy)-2-nitrophenyl)-ethylideneiminoxyacetoxy]tetrahydrofuran (mixture of isomers)

A mixture of 6.50 g of the oxime obtained in (a) above, 4.65 g of 3-acetoxy-4-(bromoacetoxy)-tetrahydrofuran, 3.10 g of potassium carbonate, 0.6 g of potassium iodide and 35 ml of acetone was refluxed with stirring for 12 hours. The resulting inorganic salts were removed by suction and the filtrate was concentrated under reduced pressure. The residue was purified by silica gel column chromatography with ethyl acetate/n-hexane (2 : 5) to give 6.6 g of the title compound (No. 34) described in Table 1 as a pale yellow viscous liquid.

IR spectrum (Cap.) $cm^{-1}$ : 1740, 1575, 1520, 1320, 1260, 1235, 1125, 1080

$^1$H-NMR spectrum ($CCl_4$-TMS) $\delta$ : 1.94, 1.99 (total 3H, each s), 2.20 (3H, s), 3.59-3.78, 3.93-4.09 (each 2H, m), 4.46, 4.65 (total 2H, each s), 5.26 (2H, m), 6.93-8.27 (6H, m)

(d) Preparation of 3-acetoxy-4-[1-(5-(2-chloro-4-trifluoromethylphenoxy)-2-nitrophenyl)-ethylideneiminoxyacetoxy]tetrahydrofuran

A mixture of 1.12 g of the compound P2 obtained in (b) above, 0.88 g of 3-acetoxy-4-(bromoacetoxy)-tetrahydrofuran, 0.50 g of potassium carbonate, 0.10 g of potassium iodide and 10 ml of acetone was refluxed with stirring for 6.5 hours. The resulting inorganic salts were removed by suction and the filtrate was concentrated under reduced pressure. The residue was purified by silica gel column chromatography with ethyl acetate/n-hexane (1 : 3) to give 0.35 g of the compound No. 35 described in Table 1 as a viscous liquid.

IR spectrum (Cap.) $cm^{-1}$ : 1740, 1570, 1520, 1315, 1260, 1235, 1125, 1075

$^1$H-NMR spectrum ($CCl_4$-TMS) $\delta$ : 1.94 (3H, s), 2.20 (3H, s), 3.67, 4.00 (each 2H, m), 4.63 (2H, s), 5.24 (2H, m), 6.90-8.13 (6H, m)

(e) Preparation of 3-acetoxy-4-[1-(5-(2-chloro-4-trifluoromethylphenoxy)-2-nitrophenyl)-ethylideneiminoxyacetoxy]tetrahydrofuran

According to the same manner as in (d) except for using the compound P3 as the starting substance, the compound No. 36 described in Table 1 was obtained as a viscous liquid.

IR spectrum (Cap.) $cm^{-1}$ : 1760, 1740, 1575, 1520, 1320, 1260, 1235, 1125, 1075

$^1$H-NMR spectrum ($CC1_4$-TMS) δ : 1.99 (3H, s), 2.18 (3H,s,), 3.69, 4.00 (each 2H, m), 4.44 (2H, s), 5.23 (2H, m), 6.90-8.24 (6H, m)

Example 9:

Preparation of methyl O-(3-acetoxy-4-tetrahydrofuryl)oxycarbonylmethyl 5-(2-chloro-4-trifluoromethylphenoxy)-2-nitrobenzohydroximate

(a) Preparation of N-[(3-acetoxy-4-tetrahydrofuryl)oxycarbonylmethoxy]phthalimide

A mixture of 13.4 g of 3-acetoxy-4-(chloroacetoxy)tetrahydrofuran, 10.96 g of potassium iodide and 50 ml of acetone was refluxed with stirring for 1 hour. After cooling to room temperature, the precipitated salts were removed by suction. The filtrate was evaporated, was then added 5 ml of N,N-dimethylformamide to the residue. Separately, a mixture of 10.28 g of N-hydroxyphthalimide, 2.64 g of pulverized sodium hydroxide and 50 ml of N,N-dimethylformamide was stirred for 1 hour at room temperature. To this mixture, was added the previously prepared N,N-dimethylformamide solution of 3-acetoxy-4-(iodoacetoxy)-tetrahydrofuran. The resultant mixture was stirred for 5 hours at room temperature and then the solvent was distilled off. The residue was treated with water to precipitate crystals. After collecting by suction, washing with water followed by drying at 55 °C for 5 hours gave 17.0 g of the title compound.
Melting point : 109 to 110.5 °C
IR spectrum (KBr) $cm^{-1}$ : 1785, 1755, 1730, 1370, 1240, 1210, 1180, 1070, 870, 695
$^1$H-NMR spectrum ($CDCl_3$-TMS) δ : 2.06 (3H, s), 3.70-4.23 (4H, m), 4.87 (2H, s), 5.33, 5.47 (each 1H, q), 7.89 (4H, m)

(b) Preparation of O-(3-acetoxy-4-tetrahydrofuryl)oxycarbonylmethyl 5-(2-chloro-4-trifluoromethylphenoxy)-2-nitrobenzohydroxamate

A mixture of 7.23 g of 5-(2-chloro-4-trifluoromethylphenoxy)-2-nitrobenzoic acid and 7.14 g of thionyl chloride was refluxed for 2 hours, and then, the excess thionyl chloride was removed to yield the acid chloride. Separate, a mixture of 7.68 g of N-[(3-acetoxy-4-tetrahydrofuryl)oxycarbonylmethoxy]phthalimide obtained in (a), 0.85 g of 100% hydrazine hydrate and 120 ml of benzene was refluxed for 2 hours, and then, cooled to room temperature followed by filtering off the precipitates. To the resultant filtrate, was added 2.26 g of pyridine, and further was added dropwise the previously prepared benzene solution of the acid chloride under cooling with water. After stirring for 4 hours at room temperature, the reaction mixture was washed twice with water followed by brine. After drying over anhydrous sodium sulfate and removal of the solvent, the residue was purified by silica gel column chromatography with ethyl acetate/n-hexane (2 : 3 to 2 : 1) to give 4.30 g of the titled compound.
Melting point : 76.5 to 76.9 °C
IR spectrum (KBr) $cm^{-1}$ : 3350, 1735, 1520, 1320, 1235, 1130, 1080
$^1$H-NMR spectrum ($CDC1_3$-TMS) δ : 2.04, 2.08 (total 3H, each s), 3.76-4.23 (4H, m), 4.66, 4.87 (total 2H, each br. s, s), 5.38 (2H, m), 7.07-8.29 (6H + ca. 0.4H, m), 9.37 (ca. 0.6H, br. s)

(c) Preparation of methyl O-(3-acetoxy-4-tetrahydrofuryl)oxycarbonylmethyl 5-(2-chloro-4-trifluoromethylphenoxy)-2-nitrobenzohydroximate

A mixture of 6.70 g of O-(3-acetoxy-4-tetrahydrofuryl)oxycarbonylmethyl 5-(2-chloro-4-trifluoromethylphenoxy)-2-nitrobenzo-hydroxamate obtained in (b), 1.31 g of potassium carbonate, 0.38 g of sodium hydroxide and 30 ml of N,N-dimethylformamide was stirred at 65 °C for 0.5 hour, and then, 2.66 g of methyl p-toluenesulfonate was added dropwise at the same temperature. After stirring for 2 hours, the solvent was removed under reduced pressure. The residue was treated with water and extracted with three portions of ether. The organic layer was washed twice with water and brine, and dried over anhydrous sodium sulfate. After evaporation, the residue was purified by silica gel column chromatography with ethyl acetate/n-hexane (2 : 3) followed by silica gel column chromatography with chloroform/2-propanol (15 : 1) to

give 1.70 g of the compound No. 40 described in Table 1 as a viscous liquid.
IR spectrum (KBr) $cm^{-1}$ : 1740, 1575, 1525, 1315, 1260, 1235, 1170, 1120, 1095, 1075
$^1H$-NMR spectrum ($CDCl_3$-TMS) $\delta$ : 2.04 (3H, s), 3.70-4.18 (4H, m), 3.93 (3H, s), 4.61 (2H, s), 5.38 (2H, m), 7.03-8.20 (6H, m)

Example 10:

Preparation of (3-acetoxy-4-tetrahydrofuryl)oxycarbonylmethyl O-methyl 5-(2-chloro-4-trifluoromethylphenoxy)-2-nitrobenzohydroximate

(a) Preparation of O-methyl 5-(2-chloro-4-trifluoromethylphenoxy)-2-nitrobenzohydroxamate

A mixture of 10.85 g of 5-(2-chloro-4-trifluoromethylphenoxy)-2-nitrobenzoic acid 8.9 g of thionyl chloride was refluxed for 2 hours, and the excess thionyl chloride was removed to yield the acid chloride.
Separately, to a solution of 5.01 g of O-methylhydroxylamine hydrochloride in 10 ml water, was added a solution of 2.88 g of sodium hydroxide in 3 ml water, with subsequent addition of 3.04 g of pyridine in 20 ml of ether. To this mixture, was added dropwise the previously prepared benzene solution of the acid chloride under cooling with water.
After stirring for 4 hours at room temperature, the reaction mixture was washed with water followed by brine. After drying over anhydrous sodium hydroxide and removal of the solvent, the residue was diluted with a small portion of ethyl acetate, followed by n-hexane to yield the crystals. By filtration, 7.15 g of the titled compound was obtained.
Melting point : 165 to 168 °C (with sublimation)
IR spectrum (KBr) $cm^{-1}$ : 3180, 1655, 1575, 1520, 1340, 1315, 1260, 1235, 1160, 1130, 1075+
$^1H$-NMR spectrum ($CDCl_3$ + DMSO-$d_6$-TMS) $\delta$ :
3.89 (3H, br. s), 7.06-8.29 (6H, m), 11.02 (1H, br. s)

(b) Preparation of (3-acetoxy-4-tetrahydrofuryl)oxycarbonylmethyl O-methyl 5-(2-chloro-4-trifluoromethylphenoxy)-2-nitrobenzohydroximate

A mixture of 2.89 g of 3-acetoxy-4-(chloroacetoxy)tetrahydrofuran, 2.16 g of potassium iodide and 15 ml of acetone was refluxed with stirring. After cooling to room temperature, the precipitated salts were filtered off and the filtrate was condensed under reduced pressure. Separately, a mixture of 3.91 g of O-methyl 5-(2-chloro-4-trifluoromethylphenoxy)-2-nitrobenzohydroxamate obtained in (a), 2.20 g of potassium carbonate and 20 ml of N,N-dimethylformamide was stirred at 64 °C for 0.5 hour. Into this mixture, was added the previously prepared 3-acetoxy-4-(iodoacetoxy)tetrahydrofuran, then, stirred for 4 hours followed by removal of the solvent under reduced pressure. The residue was treated with water and extracted with three portions of ether. The organic layer was washed with water followed by brine, and dried over anhydrous sodium sulfate. After removing the solvent, the residue was purified by silica gel column chromatography with ethyl acetate/n-hexane (1 : 2) and subsequent silica gel column chromatography with chloroform/2-propanol (30 : 1) to give 0.5 g of the compound No. 43 described in Table 1 as an amorphous solid.
IR spectrum (KBr) $cm^{-1}$ : 1745, 1575, 1525, 1320, 1260, 1235, 1170, 1125, 1085
$^1H$-NMR spectrum ($CDCl_3$-TMS) $\delta$ :
2.06, 2.07 (total 3H, each s), 3.68-4.20 (4H, m), 3.87 (3H, s), 4.93 (2H, q), 5.40 (2H, ,m), 7.03-8.18 (6H, m)

The other compounds described in Table 1 could also be prepared according to the methods of each Example No. indicated in the column titled "Preparation method". The structures of those which were prepared were identified by IR spectrum and $^1H$-NMR spectrum. The IR and $^1H$-NMR spectral data of the compounds obtained in the form of amorphous solid or viscous liquid are shown below.

Table 1

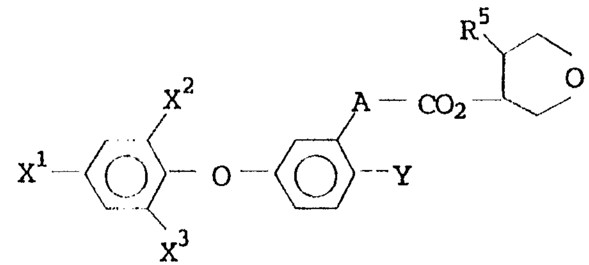

| Compound No. | $X^1$ | $X^2$ | $X^3$ | Y | A | $R^5$ | Properties | Preparation method (Example No.) |
|---|---|---|---|---|---|---|---|---|
| 1 | Cl | Cl | H | $NO_2$ | Direct bond | -OH | Amorphous solid | 1 |
| 2 | $CF_3$ | Cl | H | $NO_2$ | Direct bond | -OH | m.p. 92 - 94°C | 1 |
| 3 | $CF_3$ | Cl | F | $NO_2$ | Direct bond | -OH | | 1 |
| 4 | $CF_3$ | Cl | Cl | $NO_2$ | Direct bond | -OH | | 1 |
| 5 | $CF_3$ | Cl | H | CN | Direct bond | -OH | | 1 |
| 6 | $CF_3$ | Cl | H | Cl | Direct bond | -OH | | 1 |
| 7 | $CF_3$ | Cl | H | $CF_3$ | Direct bond | -OH | | 1 |
| 8 | $CF_3$ | Cl | H | $NO_2$ | $-CO_2CH(CH_3)-$ | -OH | | 1 |
| 9 | $CF_3$ | Cl | H | $NO_2$ | $-C(CH_3)=NOCH_2-$ | -OH | Viscous liquid | 8 |

Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 10 | $CF_3$ | Cl | H | $NO_2$ | $-C(C_2H_5)=NOCH_2-$ | -OH | | 8 |
| 11 | $CF_3$ | Cl | H | $NO_2$ | $-C(OCH_3)=NOCH_2-$ | -OH | | 9 |
| 12 | $CF_3$ | Cl | H | $NO_2$ | Direct bond | -F | $n_D^{25}$ 1.5430 | 5 |
| 13 | $CF_3$ | Cl | H | $NO_2$ | $-CO_2CH(CH_3)-$ | -F | | 5 |
| 14 | $CF_3$ | Cl | H | $NO_2$ | $-C(CH_3)=NOCH_2-$ | -F | | 5 |
| 15 | $CF_3$ | Cl | H | $NO_2$ | $-C(CH_2OCH_3)=NOCH_2-$ | -F | | 5 |
| 16 | $CF_3$ | Cl | H | $NO_2$ | $-C(CH_3)=NOCH(CH_3)-$ | -F | | 5 |
| 17 | $CF_3$ | Cl | H | $NO_2$ | $-C(OCH_3)=NOCH_2-$ | -F | | 5 |
| 18 | $CF_3$ | Cl | H | $NO_2$ | $-C(OCH_3)=NOCH(CH_3)-$ | -F | | 5 |
| 19 | $CF_3$ | Cl | H | $NO_2$ | $-C(=NOCH_3)OCH_2-$ | -F | | 5 |
| 20 | $CF_3$ | Cl | H | $NO_2$ | Direct bond | -Cl | Amorphous solid | 6 |
| 21 | $CF_3$ | Cl | H | $NO_2$ | $-CO_2CH(CH_3)-$ | -Cl | | 6 |

## Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 22 | $CF_3$ | Cℓ | H | $NO_2$ | $-C(CH_3)=NOCH_2-$ | -Cℓ | | 6 |
| 23 | $CF_3$ | Cℓ | H | $NO_2$ | $-C(OCH_3)=NOCH_2-$ | -Cℓ | | 6 |
| 24 | $CF_3$ | Cℓ | H | $NO_2$ | Direct bond | -Br | | 6 |
| 25 | $CF_3$ | Cℓ | H | $NO_2$ | Direct bond | $OCOCH_3$ | m.p. 81.5 - 82.5°C | 3 |
| 26 | $CF_3$ | Cℓ | H | $NO_2$ | Direct bond | $-OCOCH_3$ | m.p. 88 - 89°C | 3 |
| 27 | $CF_3$ | Cℓ | F | $NO_2$ | Direct bond | $-OCOCH_3$ | | 3 |
| 28 | $CF_3$ | Cℓ | Cℓ | $NO_2$ | Direct bond | $-OCOCH_3$ | | 3 |
| 29 | $CF_3$ | Cℓ | H | CN | Direct bond | $-OCOCH_3$ | | 3 |
| 30 | $CF_3$ | Cℓ | H | Cℓ | Direct bond | $-OCOCH_3$ | | 3 |
| 31 | $CF_3$ | Cℓ | H | $CF_3$ | Direct bond | $-OCOCH_3$ | | 3 |
| 32 | $CF_3$ | Cℓ | H | $NO_2$ | $-CO_2CH_2-$ | $-OCOCH_3$ | | 7 |
| 33 | $CF_3$ | Cℓ | H | $NO_2$ | $-CO_2CH(CH_3)-$ | $-OCOCH_3$ | Viscous liquid | 7 |

Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 34 | $CF_3$ | Cl | H | $NO_2$ | $-C(CH_3)=NOCH_2-$ | $-OCOCH_3$ | Viscous liquid | 8 |
| 35 | $CF_3$ | Cl | H | $NO_2$ | $-C(CH_3)=NOCH_2-$ | $-OCOCH_3$ | Viscous liquid | 8 |
| 36 | $CF_3$ | Cl | H | $NO_2$ | $-C(CH_3)=NOCH_2-$ | $-OCOCH_3$ | Viscous liquid | 8 |
| 37 | $CF_3$ | Cl | H | $NO_2$ | $-C(CH_2OCH_3)=NOCH_2-$ | $-OCOCH_3$ | | 8 |
| 38 | $CF_3$ | Cl | H | $NO_2$ | $-C(CH_3)=NOCH(CH_3)-$ | $-OCOCH_3$ | Viscous liquid | 8 |
| 39 | $CF_3$ | Cl | H | $NO_2$ | $-C(CH_3)=NOCH(C_2H_5)-$ | $-OCOCH_3$ | | 8 |
| 40 | $CF_3$ | Cl | H | $NO_2$ | $-C(OCH_3)=NOCH_2-$ | $-OCOCH_3$ | Viscous liquid | 9 |
| 41 | $CF_3$ | Cl | H | $NO_2$ | $-C(OC_2H_5)=NOCH_2-$ | $-OCOCH_3$ | | 9 |
| 42 | $CF_3$ | Cl | H | $NO_2$ | $-C(OCH_3)=NOCH(CH_3)-$ | $-OCOCH_3$ | | 9 |
| 43 | $CF_3$ | Cl | H | $NO_2$ | $-C(=NOCH_3)OCH_2-$ | $-OCOCH_3$ | Viscous liquid | 10 |
| 44 | $CF_3$ | Cl | H | $NO_2$ | $-C(=NOCH_3)OCH(CH_3)-$ | $-OCOCH_3$ | | 10 |
| 45 | Cl | Cl | H | $NO_2$ | Direct bond | $-OCOC_2H_5$ | m.p. 76.5 - 78°C | 3 |

Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 46 | $CF_3$ | Cl | H | $NO_2$ | Direct bond | $-OCOC_2H_5$ | m.p. 66.5 - 67.5°C | 3 |
| 47 | $CF_3$ | Cl | H | $NO_2$ | $-CO_2CH(CH_3)-$ | $-OCOC_2H_5$ | | 7 |
| 48 | $CF_3$ | Cl | H | $NO_2$ | $-C(CH_3)=NOCH_2-$ | $-OCOC_2H_5$ | | 8 |
| 49 | $CF_3$ | Cl | H | $NO_2$ | $-C(OCH_3)=NOCH_2-$ | $-OCOC_2H_5$ | | 9 |
| 50 | $CF_3$ | Cl | H | $NO_2$ | Direct bond | $-OCOC_3H_7$-n | Viscous liquid | 3 |
| 51 | $CF_3$ | Cl | H | $NO_2$ | Direct bond | $-OCOC_4H_9$-n | Viscous liquid | 3 |
| 52 | $CF_3$ | Cl | H | $NO_2$ | Direct bond | $-OCOCH_2Cl$ | $n_D^{25}$ 1.5507 | 3 |
| 53 | $CF_3$ | Cl | H | $NO_2$ | $-CO_2CH(CH_3)-$ | $-OCOCH_2Cl$ | | 7 |
| 54 | $CF_3$ | Cl | H | $NO_2$ | $-C(CH_3)=NOCH_2-$ | $-OCOCH_2Cl$ | | 8 |
| 55 | $CF_3$ | Cl | H | $NO_2$ | $-C(OCH_3)=NOCH_2-$ | $-OCOCH_2Cl$ | | 9 |
| 56 | $CF_3$ | Cl | H | $NO_2$ | Direct bond | $-OCOCF_3$ | | 2 |
| 57 | $CF_3$ | Cl | H | $NO_2$ | Direct bond | $-OCOCH_2OCH_3$ | m.p. 72 - 72.5°C | 2 |

## Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 58 | $CF_3$ | Cℓ | H | $NO_2$ | $-CO_2CH(CH_3)-$ | $-OCOCH_2OCH_3$ | | 7 |
| 59 | $CF_3$ | Cℓ | H | $NO_2$ | $-C(CH_3)=NOCH_2-$ | $-OCOCH_2OCH_3$ | | 8 |
| 60 | $CF_3$ | Cℓ | H | $NO_2$ | $-C(OCH_3)=NOCH_2-$ | $-OCOCH_2OCH_3$ | | 9 |
| 61 | $CF_3$ | Cℓ | H | $NO_2$ | Direct bond | $-OCOCH_2OC_2H_5$ | | 3 |
| 62 | $CF_3$ | Cℓ | H | $NO_2$ | Direct bond | $-OCOCH_2CH_2OCH_2$ | | 3 |
| 63 | $CF_3$ | Cℓ | H | $NO_2$ | Direct bond | $-OCOCH_2CO_2CH_3$ | | 3 |
| 64 | $CF_3$ | Cℓ | H | $NO_2$ | Direct bond | $-OCOCH_2CO_2C_2H_5$ | $n_D^{25}$ 1.5310 | 3 |
| 65 | $CF_3$ | Cℓ | H | $NO_2$ | Direct bond | 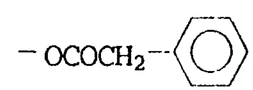 | Amorphous solid | 3 |
| 66 | $CF_3$ | Cℓ | H | $NO_2$ | Direct bond | 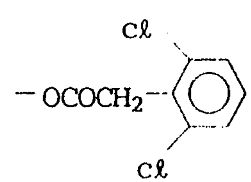 | | 3 |

Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 67 | $CF_3$ | Cℓ | H | $NO_2$ | Direct bond | 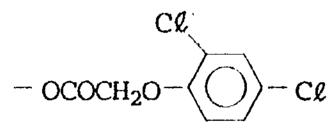 | | 3 |
| 68 | $CF_3$ | Cℓ | H | $NO_2$ | Direct bond | 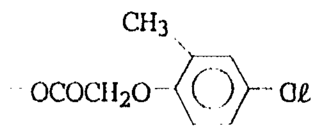 | m.p. 103 - 105°C | 3 |
| 69 | $CF_3$ | Cℓ | H | $NO_2$ | Direct bond | 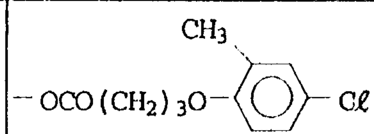 | Amorphous solid | 3 |
| 70 | $CF_3$ | Cℓ | H | $NO_2$ | Direct bond | $-OCOCH_2CH=CH_2$ | $n_D^{25}$ 1.5418 | 3 |
| 71 | $CF_3$ | Cℓ | H | $NO_2$ | Direct bond | 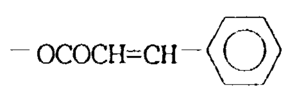 | m.p.117 - 118°C | 3 |
| 72 | $CF_3$ | Cℓ | H | $NO_2$ | Direct bond | 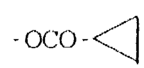 | m.p. 84.5 - 85.5°C | 3 |

Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 73 | $CF_3$ | Cℓ | H | $NO_2$ | $-CO_2CH(CH_3)-$ | -OCO- 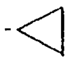 | | 7 |
| 74 | $CF_3$ | Cℓ | H | $NO_2$ | $-C(CH_3)=NOCH_2-$ | -OCO- 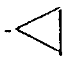 | | 8 |
| 75 | $CF_3$ | Cℓ | H | $NO_2$ | $-C(OCH_3)=NOCH_2-$ | -OCO- 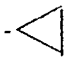 | | 9 |
| 76 | $CF_3$ | Cℓ | H | $NO_2$ | Direct bond | -OCO- 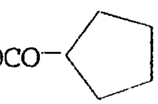 | | 3 |
| 77 | $CF_3$ | Cℓ | H | $NO_2$ | Direct bond | -OCO- 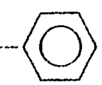 | m.p. 78 - 79.5°C | 3 |
| 78 | $CF_3$ | Cℓ | H | $NO_2$ | Direct bond | -OCO- 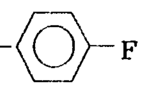 | m.p. 110.5 - 112.5°C | 3 |
| 79 | $CF_3$ | Cℓ | H | $NO_2$ | Direct bond | $-OCO_2CH_3$ | | 3 |
| 80 | Cℓ | Cℓ | H | $NO_2$ | Direct bond | $-OCO_2C_2H_5$ | Amorphous solid | 3 |
| 81 | $CF_3$ | Cℓ | H | $NO_2$ | Direct bond | $-OCON(CH_3)_2$ | | 4 |

Table 1 (cont'd)

| Compound No. | $X^1$ | $X^2$ | $X^3$ | Y | A | $R^5$ | Properties | Preparation method (Example No.) |
|---|---|---|---|---|---|---|---|---|
| 82 | $CF_3$ | Cℓ | H | $NO_2$ | Direct bond | $-OCON(CH_3)OCH_3$ | Amorphous solid | 4 |
| 83 | $CF_3$ | Cℓ | H | $NO_2$ | $-CO_2CH(CH_3)-$ | $-OCON(CH_3)OCH_3$ | | 7 |
| 84 | $CF_3$ | Cℓ | H | $NO_2$ | $-C(CH_3)=NOCH_2-$ | $-OCON(CH_3)OCH_3$ | | 8 |
| 85 | $CF_3$ | Cℓ | H | $NO_2$ | $-C(OCH_3)=NOCH_2-$ | $-OCON(CH_3)OCH_3$ | | 9 |
| 86 | $CF_3$ | Cℓ | H | $NO_2$ | Direct bond | $-OCON(CH_3)OC_2H_5$ | | 4 |
| 87 | $CF_3$ | Cℓ | H | $NO_2$ | Direct bond | $-OCON(C_2H_5)OCH_3$ | | 4 |
| 88 | $CF_3$ | Cℓ | H | $NO_2$ | Direct bond | $-OSO_2CH_3$ | Amorphous solid | 3 |
| 89 | $CF_3$ | Cℓ | H | $NO_2$ | $-CO_2CH(CH_3)-$ | $-OSO_2CH_3$ | | 7 |
| 90 | $CF_3$ | Cℓ | H | $NO_2$ | $-C(CH_3)=NOCH_2-$ | $-OSO_2CH_3$ | | 8 |
| 91 | $CF_3$ | Cℓ | H | $NO_2$ | $-C(OCH_3)=NOCH_2-$ | $-OSO_2CH_3$ | | 9 |
| 92 | $CF_3$ | Cℓ | H | $NO_2$ | Direct bond | $-OSO_2C_2H_5$ | | 9 |

| Compound No. | Spectral data |
|---|---|
| 1 | IR(Cap.)$cm^{-1}$: 3430, 1735, 1525, 1470, 1340, 1285, 1250, 1060<br>$^{1}H$-NMR($CCl_4$-TMS)δ: 2.19(1H, d, OH), 3.44-4.00 (4H, m), 4.43(1H, quint), 5.25(1H, q), 6.88-7.98(6H, m) |
| 38 | IR(Cap.)$cm^{-1}$: 1740, 1575, 1520, 1315, 1260, 1235, 1125, 1075<br>$^{1}H$-NMR($CCl_4$-TMS)δ: 1.29, 1.52(total 3H, each d), 1.90, 1.97, 1.97, 2.00 (total 3H, each s), 2.20 (3H, s), 3.60-3.75, 3.91-4.12 (each 2H, m), 4.72 (1H, q), 5.22(2H, m), 6.90-8.25(6H, m) |

| Compound No. | Spectral data |
|---|---|
| 50 | IR(Cap.)$cm^{-1}$: 1740, 1580, 1530, 1320, 1280, 1260, 1170, 1130, 1075<br>$^1$H-NMR($CCl_4$-TMS)δ: 0.88(3H, t), 1.56(2H, sext), 2.22(2H, t), 3.59-4.21(4H, m), 5.27, 5.47(each 1H, q), 6.97-8.04(6H,m) |
| 51 | IR(Cap.)$cm^{-1}$: 1740, 1580, 1530, 1320, 1280, 1260, 1170, 1130, 1075<br>$^1$H-NMR $CCl_4$-TMS)δ: 0.87(3H, t), 1.36(4H, m), 2.24(2H, t), 3.59-4.21(4H, m), 5.27, 5.47(each 1H, q), 6.96-8.04(6H, m) |

| Compound No. | Spectral data |
|---|---|
| 65 | IR(Cap.)$cm^{-1}$: 1745, 1580, 1530, 1320, 1285, 1260, 1130, 1080<br>$^1H$-NMR($CCl_4$-TMS)δ: 3.52(2H, s), 3.74, 4.07 (each 2H, m), 5.28, 5.46 (each 1H, q), 6.88-8.01 (11H, m) |
| 69 | IR(KBr)$cm^{-1}$: 1740, 1580, 1520, 1490, 1315, 1285, 1250, 1125<br>$^1H$-NMR($CCl_4$-TMS)δ: 2.07(2H, m), 2.12(3H, s), 2.50(2H, m), 3.60-4.21(6H, m), 5.30, 5.47(each 1H, q), 6.54-7.94(9H, m) |

| Compound No. | Spectral data |
|---|---|
| 80 | IR(Cap.)$cm^{-1}$: 1750, 1530, 1470, 1340, 1285, 1250<br>$^1H$-NMR($CCl_4$-TMS)δ: 1.24(3H, t), 3.68-4.22(6H, m), 5.20, 5.50(each 1H, q), 6.86-8.05(6H, m) |
| 88 | IR(Cap.)$cm^{-1}$: 1740, 1580, 1525, 1340, 1320, 1280, 1260, 1170, 1130, 1075<br>$^1H$-NMR($CCl_4$-TMS)δ: 2.91(3H, s), 3.74-4.23(4H, m), 5.16, 5.45(each 1H, q), 7.07-8.12(6H, m) |

Shown below are the formulation examples of the compounds of the present invention. In the following, all "parts" and "%" are by weight unless otherwise noted.

Formulation Example 1: Wettable powder

40 parts of each compound of this invention shown in Table 1, 20 parts of Carplex #80 (trade mark, mfd. by Shionogi Seiyaku Co., Ltd.), 35 parts of N,N-Kaolin Clay (trade mark, mfd. by Tsuchiya Kaolin Co., Ltd.) and 5 parts of Sorpol 8070 (trade mark, a higher alcohol sulfate type surfactant, mfd. by Toho Kagaku Co., Ltd.) were uniformly mixed and ground to obtain a wettable powder containing 40% of the active ingredient.

Formulation Example 2: Emulsifiable concentrate

20 parts of each compound of this invention shown in Table 1 was dissolved in a mixed solvent consisting of 35 parts of xylene and 30 parts of dimethylformamide. To this solution, was added 15 parts of Sorpol 3005X (trade mark, a polyoxyethylene type surfactant, mfd. by Toho Kagaku Co., Ltd.) to obtain an emulsifiable concentrate containing 20% of the active ingredient.

Formulation Example 3: Flowable agent

30 parts of each compound of this invention shown in Table 1 was well mixed and dispersed in a previously prepared mixture of 8 parts of ethylene glycol, 5 parts of Sorpol AC-3020 (trade mark, mfd. by Toho Kagaku Co., Ltd.), 0.1 part of xanthan gum and 56.9 parts of water to form a slurry mixture. This slurry mixture was wet pulverized by Dyno-Mill (trade mark, mfd. by Simmal Enterprizes Co., Ltd.) to obtain a stable flowable agent containing 30% of the active ingredient.

Formulation Example 4: Granules

3 parts of each compound of this invention shown in Table 1, 41 parts of clay (mfd. by Nippon Talc Co., Ltd.), 55 parts of bentonite (mfd. by Hojun Yoko Co., Ltd.) and 1 part of Aerol CT-1 (trade mark, a succinate type surfactant, mfd. by Toho Kagaku Co., Ltd.) were mixed and ground, and the mixture was kneaded after adding 20 parts of water. This mixture was extruded from 0.6 mm-diameter nozzle by using a pelletizer, dried at 60 °C for 2 hours and then cut to a length of 1 to 2 mm to obtain granules containing 3% of the active ingredient.

Test Example 1: Foliage treatment

Volcanic ash soil from upland field was placed in a small expanded polystyrene pots having an area of 110 $cm^2$, and after fertilization, the seeds of Amaranthus retroflexus, Persicaria blumei gross, Abutilon avicennae, Xanthium strumarium L., Galium aparine, Viola tricolor, corn, wheat and soybean were sown in the respective ports.

The pots were kept in a greenhouse for cultivation, and when the test plants have grown to the predetermined leaf stages (L), that is, 3L for Amaranthus retroflexus, 3L for Persicaria blumei gross, 2L for Abutilon avicennae, 2L of Xanthium strumarium L., 2L for Galium aparine, 3L for Viola tricolor, 3.5L for corn, 3L for wheat and 1.5L for soybean, each wettable powder obtained in Formulation Example 1 and the wettable powders obtained in the same manner as in Formulation Example 1 using as the active ingredient each of the references, sodium 5-(2-chloro-4-trifluoromethylphenoxy)-2-nitrobenzoate (A), methyl 5-(2-chloro-4-trifluoromethylphenoxy)-2-nitroacetophenoneoxime-O-acetate (B) and 2-chloro-4-ethylamino-6-isopropylamino-1,3,5-triazine (C) were sprayed on leaves and stems of the test plant by a small pressure sprayer in an amount of about 500 litres after diluting each wettable powder with water so that the application amount of the active ingredient was 1.0 kg, 0.5 kg, 0.25 kg, 0.125 kg and 0.0625 kg per ha.

Thereafter, cultivation of the plants was continued in the greenhouse, and on the 21st day after the treatment, the herbicidal effect and phytotoxicity against crops by the compounds were examined.

The results are shown in Table 2.

The herbicidal effect was evaluated by determining the value (Y, %) from the following formula:

$$\left(1 - \frac{\text{Fresh weight of aerial part of weeds in treated pot}}{\text{Fresh weight of aerial part of weeds in control pot}}\right) \times 100 = Y\ (\%)$$

and expressed by the herbicidal index according to the following ratings:

| Herbicidal index | Y (%) |
|---|---|
| 0 | 0~5 |
| 1 | 6~30 |
| 2 | 31~50 |
| 3 | 51~70 |
| 4 | 71~90 |
| 5 | 91~100 |

The evaluation of phytotoxicity to crops by the compounds was made by calculating the value of Y′ from the following formula:

$$\left( 1 - \frac{\text{Fresh weight of aerial part of crop in treated pot}}{\text{Fresh weight of aerial part of crop in control pot}} \right) \times 100 = Y' (\%)$$

and expressed by phytotoxicity index according to the following ratings:

| Phytotoxicity index | Y' (%) |
|---|---|
| 0 | 0~5 |
| 1 | 6~10 |
| 2 | 11~20 |
| 3 | 21~40 |
| 4 | 41~60 |
| 5 | 61~100 |

Table 2

| Compound No. | Application dosage kg/ha | Herbicidal effect | | | | | | Phytotoxicity | | |
|---|---|---|---|---|---|---|---|---|---|---|
| | | *Amaranthus retroflexus* | *Persicaria blumei gross* | *Abutilon avicennae* | *Xanthium strumarium* L. | *Galium aparine* | *Viola tri-color* | Corn | Wheat | Soybean |
| 1 | 0.5 | 5 | 5 | 5 | 5 | 4 | 5 | 0 | 0 | 0 |
| | 0.25 | 5 | 5 | 4 | 4 | 2 | 4 | 0 | 0 | 0 |
| 2 | 0.25 | 5 | 5 | 5 | 5 | 5 | 5 | 1 | 1 | 2 |
| | 0.125 | 5 | 5 | 5 | 5 | 5 | 5 | 0 | 0 | 0 |
| 9 | 0.125 | 5 | 5 | 5 | 5 | 5 | 5 | 1 | 1 | 1 |
| | 0.0625 | 5 | 5 | 5 | 5 | 5 | 5 | 0 | 0 | 0 |
| 12 | 0.25 | 5 | 5 | 5 | 5 | 5 | 5 | 0 | 1 | 1 |
| | 0.125 | 5 | 5 | 5 | 4 | 3 | 5 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | *Amaranthus retroflexus* | *Persicaria blumei gross* | *Abutilon avicennae* | *Xanthium strumarium* L. | *Galium aparine* | *Viola tri-color* | Corn | Wheat | Soybean |
| 20 | 0.25 | 5 | 5 | 5 | 5 | 5 | 5 | 0 | 1 | 1 |
| | 0.125 | 5 | 5 | 5 | 4 | 4 | 5 | 0 | 0 | 0 |
| 25 | 0.5 | 5 | 5 | 5 | 5 | 5 | 4 | 0 | 0 | 0 |
| | 0.25 | 5 | 5 | 5 | 4 | 4 | 3 | 0 | 0 | 0 |
| 26 | 0.25 | 5 | 5 | 5 | 5 | 5 | 5 | 1 | 0 | 2 |
| | 0.125 | 5 | 5 | 5 | 5 | 5 | 5 | 0 | 0 | 0 |
| 33 | 0.25 | 5 | 5 | 5 | 5 | 5 | 5 | 2 | 1 | 1 |
| | 0.125 | 5 | 5 | 5 | 5 | 5 | 5 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | *Amaranthus retroflexus* | *Persicaria blumei gross* | *Abutilon avicennae* | *Xanthium strumarium* L. | *Galium aparine* | *Viola tri-color* | Corn | Wheat | Soybean |
| 34 | 0.125 | 5 | 5 | 5 | 5 | 5 | 5 | 1 | 1 | 1 |
| | 0.0625 | 5 | 5 | 5 | 5 | 5 | 5 | 0 | 1 | 0 |
| 35 | 0.125 | 5 | 5 | 5 | 5 | 5 | 5 | 1 | 1 | 2 |
| | 0.0625 | 5 | 5 | 5 | 5 | 5 | 5 | 0 | 0 | 0 |
| 36 | 0.125 | 5 | 5 | 5 | 5 | 5 | 5 | 0 | 1 | 1 |
| | 0.0625 | 5 | 5 | 5 | 5 | 5 | 5 | 0 | 0 | 1 |
| 38 | 0.125 | 5 | 5 | 5 | 5 | 5 | 5 | 1 | 1 | 1 |
| | 0.0625 | 5 | 5 | 5 | 5 | 5 | 5 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | *Amaranthus retroflexus* | *Persicaria blumei gross* | *Abutilon avicennae* | *Xanthium strumarium L.* | *Galium aparine* | *Viola tri-color* | Corn | Wheat | Soybean |
| 40 | 0.125 | 5 | 5 | 5 | 5 | 5 | 5 | 1 | 1 | 2 |
| | 0.0625 | 5 | 5 | 5 | 5 | 5 | 5 | 0 | 1 | 0 |
| 43 | 0.125 | 5 | 5 | 5 | 5 | 5 | 5 | 0 | 1 | 1 |
| | 0.0625 | 5 | 5 | 5 | 4 | 3 | 5 | 0 | 0 | 0 |
| 45 | 0.5 | 5 | 5 | 5 | 5 | 4 | 5 | 0 | 0 | 1 |
| | 0.25 | 5 | 5 | 5 | 4 | 4 | 3 | 0 | 0 | 0 |
| 46 | 0.25 | 5 | 5 | 5 | 5 | 5 | 5 | 1 | 0 | 2 |
| | 0.125 | 5 | 5 | 5 | 5 | 5 | 5 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | Amaranthus retroflexus | Persicaria blumei gross | Abutilon avicennae | Xanthium strumarium L. | Galium aparine | Viola tri-color | Corn | Wheat | Soybean |
| 50 | 0.25 | 5 | 5 | 5 | 5 | 4 | 5 | 0 | 1 | 1 |
| | 0.125 | 5 | 5 | 5 | 4 | 4 | 5 | 0 | 0 | 0 |
| 51 | 0.25 | 5 | 5 | 5 | 5 | 4 | 5 | 0 | 0 | 1 |
| | 0.125 | 5 | 5 | 5 | 4 | 3 | 5 | 0 | 0 | 0 |
| 52 | 0.25 | 5 | 5 | 5 | 5 | 5 | 5 | 0 | 0 | 1 |
| | 0.125 | 5 | 5 | 5 | 5 | 3 | 5 | 0 | 0 | 0 |
| 57 | 0.25 | 5 | 5 | 5 | 5 | 5 | 5 | 1 | 1 | 1 |
| | 0.125 | 5 | 5 | 5 | 5 | 5 | 5 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | Amaranthus retroflexus | Persicaria blumei, gross | Abutilon avicennae | Xanthium strumarium L. | Galium aparine | Viola tri-color | Corn | Wheat | Soybean |
| 64 | 0.25 | 5 | 5 | 5 | 5 | 5 | 5 | 1 | 0 | 1 |
| | 0.125 | 5 | 5 | 5 | 5 | 3 | 5 | 0 | 0 | 0 |
| 65 | 0.25 | 5 | 5 | 5 | 5 | 4 | 5 | 0 | 0 | 0 |
| | 0.125 | 5 | 5 | 5 | 4 | 3 | 4 | 0 | 0 | 0 |
| 68 | 0.5 | 5 | 5 | 5 | 5 | 4 | 5 | 0 | 0 | 0 |
| | 0.25 | 5 | 4 | 5 | 4 | 3 | 4 | 0 | 0 | 0 |
| 69 | 0.5 | 5 | 5 | 5 | 5 | 5 | 5 | 0 | 0 | 0 |
| | 0.25 | 5 | 5 | 5 | 4 | 3 | 4 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | Amaranthus retroflexus | Persicaria blumei gross | Abutilon avicennae | Xanthium strumarium L. | Galium aparine | Viola tri-color | Corn | Wheat | Soybean |
| 70 | 0.25 | 5 | 5 | 5 | 5 | 4 | 5 | 1 | 0 | 1 |
| | 0.125 | 5 | 5 | 5 | 4 | 4 | 5 | 0 | 0 | 0 |
| 71 | 0.5 | 5 | 5 | 5 | 4 | 4 | 5 | 0 | 0 | 0 |
| | 0.25 | 5 | 4 | 5 | 4 | 3 | 4 | 0 | 0 | 0 |
| 72 | 0.25 | 5 | 5 | 5 | 5 | 5 | 5 | 0 | 0 | 1 |
| | 0.125 | 5 | 5 | 5 | 5 | 4 | 5 | 0 | 0 | 0 |
| 77 | 0.5 | 5 | 5 | 5 | 5 | 4 | 5 | 0 | 0 | 1 |
| | 0.25 | 5 | 5 | 5 | 5 | 3 | 4 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | *Amaranthus retroflexus* | *Persicaria blumei gross* | *Abutilon avicennae* | *Xanthium strumarium L.* | *Galium aparine* | *Viola tricolor* | Corn | Wheat | Soybean |
| 78 | 0.5 | 5 | 5 | 5 | 5 | 4 | 5 | 1 | 0 | 1 |
| | 0.25 | 5 | 5 | 5 | 5 | 3 | 4 | 0 | 0 | 0 |
| 80 | 0.5 | 5 | 5 | 5 | 5 | 4 | 5 | 0 | 0 | 0 |
| | 0.25 | 5 | 5 | 4 | 4 | 3 | 4 | 0 | 0 | 0 |
| 82 | 0.5 | 5 | 5 | 5 | 5 | 5 | 5 | 0 | 0 | 1 |
| | 0.25 | 5 | 5 | 5 | 4 | 3 | 3 | 0 | 0 | 0 |
| 88 | 0.5 | 5 | 5 | 5 | 5 | 4 | 5 | 0 | 0 | 0 |
| | 0.25 | 5 | 5 | 4 | 3 | 3 | 3 | 0 | 0 | 0 |

Table 2 (cont'd)

| Compound No. | Application dosage kg/ha | Herbicidal effect | | | | | | Phytotoxicity | | |
|---|---|---|---|---|---|---|---|---|---|---|
| | | *Amaranthus retroflexus* | *Persicaria blumei gross* | *Abutilon avicennae* | *Xanthium strumarium* L. | *Galium aparine* | *Viola tri-color* | Corn | Wheat | Soybean |
| Reference (A) | 0.5 | 5 | 5 | 3 | 4 | 4 | 5 | 3 | 3 | 2 |
| | 0.25 | 5 | 5 | 1 | 3 | 2 | 4 | 1 | 1 | 1 |
| Reference (B) | 0.25 | 5 | 5 | 5 | 5 | 5 | 5 | 5 | 5 | 5 |
| | 0.125 | 5 | 5 | 5 | 4 | 5 | 5 | 5 | 4 | 5 |
| Reference (C) | 1.0 | 5 | 5 | 2 | 3 | 3 | 5 | 0 | 3 | 5 |
| | 0.5 | 5 | 4 | 0 | 1 | 2 | 3 | 0 | 1 | 3 |
| Control | - | 0 | 0 | 0 | 0 | 0 | 0 | 0 | 0 | 0 |

Test Example 2: Upland soil treatment

Volcanic ash soil from upland field was placed in a resin vat having an area of 600 $cm^2$, and after fertilization, the seeds of corn, wheat and soybean were sown and covered with soil to a depth of 2 to 3 cm. Further, the soil containing the seeds of weed, Digitaria adscendens, Chenopodium album L., Persicaria blumei gross, Abutilon avicennae and Bidens pilosa was placed thereon uniformly. Each of the wettable powders obtained in Formulation Example 1 and the wettable powders containing each of the references, (A) and (B) in Test Example 1 as the active ingredient, which were obtained in the same manner as in Formulation Example 1, were diluted with water and sprayed on the soil surface by a small pressure sprayer so that the application amount of the active ingredient was 1.0 kg, 0.5 kg, 0.25 kg, or 0.125 kg per ha.

Thereafter, the cultivation of the test plant was allowed to continue in a greenhouse. On the 21st day after the treatment, the herbicidal effect and phytotoxicity to the crops of the compounds were examined and evaluated according to the same ratings as in Test Example 1. The results are shown in Table 3.

Table 3

| Compound No. | Application dosage kg/ha | Herbicidal effect | | | | | Phytotoxicity | | |
|---|---|---|---|---|---|---|---|---|---|
| | | Digitaria adscendens | Chenopodium album L. | Persicaria blumei gross | Abutilon avicennae | Bidens pilosa | Corn | Wheat | Soybean |
| 9 | 0.5 | 5 | 5 | 5 | 5 | 5 | 0 | 1 | 0 |
| | 0.25 | 5 | 5 | 5 | 5 | 5 | 0 | 0 | 0 |
| 12 | 0.5 | 5 | 5 | 5 | 5 | 5 | 1 | 0 | 1 |
| | 0.25 | 5 | 5 | 5 | 5 | 4 | 0 | 0 | 0 |
| 26 | 0.5 | 5 | 5 | 5 | 5 | 5 | 0 | 1 | 1 |
| | 0.25 | 5 | 5 | 5 | 5 | 5 | 0 | 0 | 0 |
| 33 | 0.5 | 5 | 5 | 5 | 5 | 5 | 0 | 0 | 0 |
| | 0.25 | 4 | 5 | 4 | 4 | 5 | 0 | 0 | 0 |

Table 3 (cont'd)

| Compound No. | Application dosage kg/ha | Herbicidal effect | | | | | Phytotoxicity | | |
|---|---|---|---|---|---|---|---|---|---|
| | | Digitaria adscendens | Chenopodium album L. | Persicaria blumei gross | Abutilon avicennae | Bidens pilosa | Corn | Wheat | Soybean |
| 34 | 0.5 | 5 | 5 | 5 | 5 | 5 | 0 | 1 | 0 |
| | 0.25 | 5 | 5 | 5 | 5 | 5 | 0 | 0 | 0 |
| 35 | 0.5 | 5 | 5 | 5 | 5 | 5 | 1 | 1 | 1 |
| | 0.25 | 5 | 5 | 5 | 5 | 5 | 0 | 0 | 0 |
| 36 | 0.5 | 5 | 5 | 5 | 5 | 5 | 0 | 1 | 1 |
| | 0.25 | 5 | 5 | 5 | 5 | 5 | 0 | 0 | 0 |
| 38 | 0.5 | 5 | 5 | 5 | 5 | 5 | 0 | 0 | 0 |
| | 0.25 | 5 | 5 | 5 | 5 | 5 | 0 | 0 | 0 |

Table 3 (cont'd)

| Compound No. | Application dosage kg/ha | Herbicidal effect | | | | | Phytotoxicity | | |
|---|---|---|---|---|---|---|---|---|---|
| | | *Digitaria adscendens* | *Chenopodium album* L. | *Persicaria blumei gross* | *Abutilon avicennae* | *Bidens pilosa* | Corn | Wheat | Soybean |
| 40 | 0.5 | 5 | 5 | 5 | 5 | 5 | 0 | 0 | 1 |
| | 0.25 | 5 | 5 | 5 | 5 | 5 | 0 | 0 | 0 |
| 43 | 0.5 | 5 | 5 | 5 | 5 | 5 | 0 | 0 | 0 |
| | 0.25 | 4 | 5 | 5 | 5 | 4 | 0 | 0 | 0 |
| 46 | 0.5 | 5 | 5 | 5 | 5 | 5 | 1 | 0 | 2 |
| | 0.25 | 5 | 5 | 5 | 5 | 5 | 0 | 0 | 0 |
| 50 | 0.5 | 5 | 5 | 5 | 5 | 5 | 0 | 0 | 1 |
| | 0.25 | 5 | 5 | 5 | 5 | 5 | 0 | 0 | 0 |

Table 3 (cont'd)

| Compound No. | Application dosage kg/ha | Herbicidal effect | | | | | Phytotoxicity | | |
|---|---|---|---|---|---|---|---|---|---|
| | | *Digitaria adscendens* | *Chenopodium album* L. | *Persicaria blumei gross* | *Abutilon avicennae* | *Bidens pilosa* | Corn | Wheat | Soybean |
| 52 | 0.5 | 5 | 5 | 5 | 5 | 5 | 0 | 1 | 1 |
| | 0.25 | 5 | 5 | 5 | 5 | 5 | 0 | 0 | 0 |
| 57 | 0.5 | 5 | 5 | 5 | 5 | 5 | 0 | 0 | 0 |
| | 0.25 | 5 | 4 | 5 | 4 | 4 | 0 | 0 | 0 |
| 64 | 0.5 | 5 | 5 | 5 | 5 | 5 | 0 | 0 | 0 |
| | 0.25 | 3 | 5 | 5 | 4 | 5 | 0 | 0 | 0 |
| 70 | 0.5 | 5 | 5 | 5 | 5 | 5 | 0 | 0 | 1 |
| | 0.25 | 5 | 5 | 3 | 4 | 4 | 0 | 0 | 0 |

Table 3 (cont'd)

| Compound No. | Application dosage kg/ha | Herbicidal effect | | | | | Phytotoxicity | | |
|---|---|---|---|---|---|---|---|---|---|
| | | Digitaria adscendens | Chenopodium album L. | Persicaria blumei gross | Abutilon avicennae | Bidens pilosa | Corn | Wheat | Soybean |
| Reference (A) | 1.0 | 4 | 5 | 5 | 2 | 4 | 2 | 2 | 1 |
| | 0.5 | 3 | 4 | 3 | 1 | 3 | 1 | 0 | 0 |
| Reference (B) | 0.5 | 5 | 5 | 5 | 5 | 5 | 5 | 5 | 5 |
| | 0.25 | 5 | 5 | 5 | 4 | 4 | 4 | 4 | 5 |
| Control | - | 0 | 0 | 0 | 0 | 0 | 0 | 0 | 0 |

Test Example 3: Soil treatment in paddy condition

Alluvial clay loam at paddy field was placed in a resin vat (400 $cm^2$). After fertilization, a suitable amount of water was added and the soil was puddled. The seeds of weeds Echinochloa crus-galli var. hispidula, Monochoria vaginalis and Scirpus juncoides were sown in the soil layer within 0.5 cm from the soil surface. Then the tubers of Cyperus serotinus were planted in the soil surface (4 tubers per vat).

Further, the rice seedlings (variety: Akinishiki) of 2.1-foliar stage were transplanted into the vat (2 sets of seedlings per vat; one set consisting of three seedlings), and then the vat was filled with water. The depth of water above the soil surface was kept at about 3.5 cm.

On the 5th day after transplantation of rice seedlings, each granules in Formulation Example 4 and the granules containing the references A and B as active ingredient obtained in the same manner as in Formulation Example 1 were sprinkle over the water surface so that the application amounts of the active ingredient was 1.0 kg/ha, 0.5 kg/ha and 0.25 kg/ha.

After the treatment, leakage of water was effected for two days by 3 cm/day, and thereafter the seedlings were allowed to grow in a greenhouse.

On the 21st day after the treatment, the herbicidal effect and phytotoxicity to the transplanted rice plants were examined and evaluated according to the same ratings as in Test Example 1. The results are shown in Table 4.

Table 4

| Compound No. | Application dosage kg/ha | Herbicidal effect | | | | Phytotoxicity |
|---|---|---|---|---|---|---|
| | | *Echinochloa crus-galli* var. hispidula | *Monochoria vaginalis* | *Scirpus juncoides* | *Cyperus serotinus* | Transplanted rice plant |
| 2 | 0.5 | 5 | 5 | 5 | 5 | 1 |
| | 0.25 | 5 | 5 | 5 | 5 | 0 |
| 9 | 0.25 | 5 | 5 | 5 | 5 | 2 |
| | 0.125 | 5 | 5 | 5 | 5 | 0 |
| 12 | 0.5 | 5 | 5 | 5 | 5 | 1 |
| | 0.25 | 5 | 5 | 5 | 5 | 0 |
| 26 | 0.5 | 5 | 5 | 5 | 5 | 0 |
| | 0.25 | 5 | 5 | 5 | 5 | 0 |
| 34 | 0.25 | 5 | 5 | 5 | 5 | 1 |
| | 0.125 | 5 | 5 | 5 | 5 | 0 |

Table 4 (cont'd)

| Compound No. | Application dosage kg/ha | Herbicidal effect | | | | Phytotoxicity |
|---|---|---|---|---|---|---|
| | | *Echinochloa crus-galli* var. hispidula | *Monochoria vaginalis* | *Scirpus juncoides* | *Cyperus serotinus* | Transplanted rice plant |
| 35 | 0.25 | 5 | 5 | 5 | 5 | 0 |
| | 0.125 | 5 | 5 | 3 | 5 | 0 |
| 36 | 0.25 | 5 | 5 | 5 | 5 | 0 |
| | 0.125 | 5 | 5 | 3 | 5 | 0 |
| 38 | 0.25 | 5 | 5 | 5 | 5 | 2 |
| | 0.125 | 5 | 5 | 5 | 5 | 0 |
| 40 | 0.25 | 5 | 5 | 5 | 5 | 1 |
| | 0.125 | 5 | 5 | 5 | 5 | 0 |
| 43 | 0.25 | 5 | 5 | 5 | 5 | 0 |
| | 0.125 | 5 | 5 | 4 | 5 | 0 |

Table 4 (cont'd)

| Compound No. | Application dosage kg/ha | Herbicidal effect | | | | Phytotoxicity |
|---|---|---|---|---|---|---|
| | | *Echinochloa crus-galli* var. hispidula | *Monochoria vaginalis* | *Scirpus juncoides* | *Cyperus serotinus* | Transplanted rice plant |
| 46 | 0.5 | 5 | 5 | 5 | 5 | 1 |
| | 0.25 | 5 | 5 | 5 | 5 | 0 |
| 50 | 0.5 | 5 | 5 | 5 | 5 | 0 |
| | 0.25 | 5 | 5 | 5 | 5 | 0 |
| 51 | 0.5 | 5 | 5 | 5 | 5 | 0 |
| | 0.25 | 5 | 5 | 5 | 5 | 0 |
| 52 | 0.5 | 5 | 5 | 5 | 5 | 1 |
| | 0.25 | 5 | 5 | 5 | 5 | 0 |
| 57 | 0.5 | 5 | 5 | 5 | 5 | 2 |
| | 0.25 | 5 | 5 | 5 | 5 | 0 |

Table 4 (cont'd)

| Compound No. | Application dosage kg/ha | Herbicidal effect | | | | Phytotoxicity |
|---|---|---|---|---|---|---|
| | | *Echinochloa crus-galli* var. hispidula | *Monochoria vaginalis* | *Scirpus juncoides* | *Cyperus serotinus* | Transplanted rice plant |
| 72 | 0.5 | 5 | 5 | 5 | 5 | 0 |
| | 0.25 | 4 | 5 | 4 | 5 | 0 |
| Reference (A) | 1.0 | 5 | 5 | 4 | 5 | 2 |
| | 0.5 | 4 | 4 | 1 | 2 | 0 |
| Reference (B) | 0.5 | 5 | 5 | 5 | 5 | 5 |
| | 0.25 | 4 | 5 | 5 | 5 | 4 |
| Control | - | 0 | 0 | 0 | 0 | 0 |

## Claims

1. A diphenyl ether derivative represented by the formula (I):

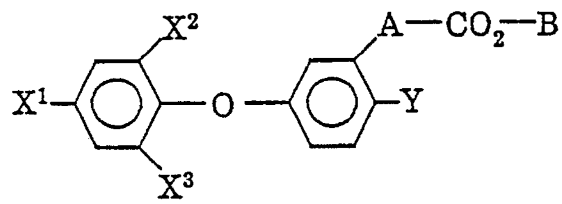

( I )

wherein $X^1$ represents a halogen atom or a $C_1$-$C_3$ haloalkyl group; $X^2$ and $X^3$ represent independently a hydrogen atom, a halogen atom, a trifluromethyl group, a nitro group or a cyano group; Y represents a nitro group, a cyano group, a halogen atom, a $C_1$-$C_3$ haloalkyl group or a hydrogen atom; A represents a direct bond, a group represented by the formula:
$-CO_2(CH(R^1)-$
wherein $R^1$ represents a hydrogen atom or a $C_1$-$C_3$ alkyl group, a group represented by the formula:
$-C(R^2) = NOCH(R^3)-$
wherein $R^2$ represents a hydrogen atom, a $C_1$-$C_4$ alkyl group which may be substituted with a $C_1$-$C_4$ alkoxy group, $C_1$-$C_4$ alkylthio group or halogen atom, or a $C_1$-$C_3$ alkoxy group, and $R^3$ represents a hydrogen atom or a $C_1$-$C_3$ alkyl group, or a group represented by the formula:
$-C(=NOR^4)OCH(R^3)-$
wherein $R^3$ is the same as defined above and $R^4$ represents a $C_1$-$C_3$ alkyl group; and B represents a group represented by the formula:

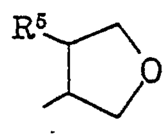

wherein $R^5$ represents a halogen atom; a hydroxyl group; an acyloxy group having up to 6 carbon atoms which may be substituted with a halogen atom, $C_1$-$C_6$ alkoxy group, $C_2$-$C_7$ alkoxycarbonyl group, aryl group which may be substituted with 1 to 3 halogen atoms and/or 1 to 3 $C_1$-$C_3$ alkyl groups or aryloxy group which may be substituted with 1 to 3 halogen atoms and/or 1 to 3 $C_1$-$C_3$ alkyl groups; a $C_4$-$C_9$ cycloalkylcarbonyloxy group; a benzoyloxy group which may be substituted with a halogen atom, $C_1$-$C_4$ alkyl group or $C_1$-$C_4$ alkoxy group; a $C_2$-$C_7$ alkoxycarbonyloxy group; a $C_1$-$C_4$ alkylsufonyloxy group; or a carbamoyloxy group which may be substituted with a $C_1$-$C_4$ alkyl group and/or $C_1$-$C_4$ alkoxy group.

2. A diphenyl ether derivative represented by the formula (I′):

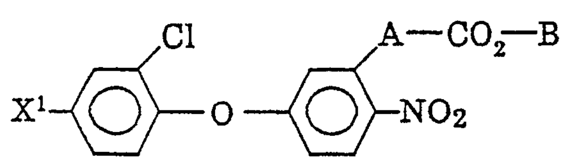

( I ')

wherein $X^1$ represents a halogen atom or a trifluoromethyl group; A represents a direct bond, a group represented by the formula:
$-CO_2CH(R^1)-$
wherein $R^1$ represents a hydrogen atom or a $C_1$-$C_3$ alkyl group, or a group represented by the formula:
$-C(R^2) = NOCH(R^3)-$
wherein $R^2$ represents a hydrogen atom, a $C_1$-$C_3$ alkyl group which may be substituted with a methoxy group or a $C_1$-$C_3$ alkoxy group, and $R^3$ represents a hydrogen atom or a $C_1$-$C_3$ alkyl group, or a group

represented by the formula:
-C(=NOR[4])OCH(R[3])-
wherein $R^3$ is the same as defined above and $R^4$ represents a $C_1$-$C_3$ alkyl group; and B represents a group represented by the formula:

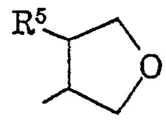

wherein $R^5$ represents a halogen atom; a hydroxyl group; a $C_2$-$C_4$ acyloxy group being saturated or having one double bond which may be substituted with a halogen atom, $C_1$-$C_3$ alkoxy group, $C_2$-$C_5$ alkoxycarbonyl group, phenyl group which may be substituted with 1 to 3 halogen atoms and/or 1 to 3 $C_1$-$C_3$ alkyl groups or phenoxy group which may be substituted with 1 to 3 halogen atoms and/or 1 to 3 $C_1$-$C_3$ alkyl groups; a $C_4$-$C_7$ cycloalkylcarbonyloxy group; a benzoyloxy group which may be substituted with a halogen atom; a $C_2$-$C_5$ alkoxycarbonyloxy group; a $C_1$-$C_2$ alkylsufonyloxy group; or a carbamoyloxy group which may be substituted with a $C_1$-$C_2$ alkyl group and/or $C_1$-$C_2$ alkoxy group.

3. A herbicidal composition comprising a herbicidally effective amount of the diphenyl ether derivative according to claim 1 and a herbicidally acceptable adjuvant, carrier or surfactant.

4. A herbicidal composition comprising a herbicidally effective amount of the diphenyl ether derivative according to claim 2 and a herbicidally acceptable adjuvant, carrier or surfactant.

5. A process for producing the diphenyl ether derivative according to claim 1, which comprises reacting a compound represented by the formula (II):

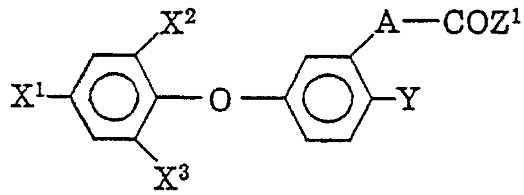

(II)

wherein $Z^1$ represents a halogen atom and $X^1$, $X^2$, $X^3$, A and Y are the same as defined in claim 1, with a compound represented by the formula:
HO-B
wherein B is the same as defined in claim 1,
in the presence of absence of a base at a temperature from -30 to 120 °C.

6. A process for producing the diphenyl ether derivative represented by the following formula (Ib):

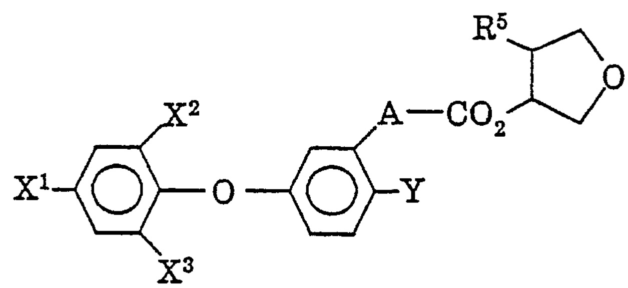

(I b)

wherein $R^5$ is the same as defined in claim 1 except for a halogen atom and hydroxyl group, and $X^1$, $X^2$, $X^3$, A and Y are the same as defined in claim 1,
which comprises reacting a compound represented by the formula (Ia):

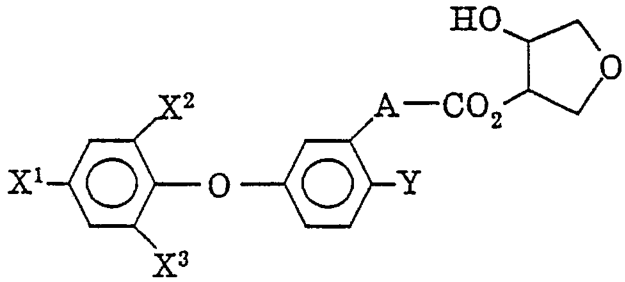

( I a)

wherein $X^1$, $X^2$, $X^3$, A and Y are the same as defined in claim 1, with a compound of the formula:
$Z^2$-$R^{52}$
wherein $R^{52}$ represents a $C_2$-$C_6$ acyl group which may be substituted with one selected from the group consisting of a halogen atom, a $C_1$-$C_6$ alkoxy group, a $C_2$-$C_7$ alkoxycarbonyl group, an aryl group which may be substituted with 1 to 3 halogen atoms and/or 1 to 3 $C_1$-$C_3$ alkyl groups and an aryloxy group which may be substituted with 1 to 3 halogen atoms and/or 1 to 3 $C_1$-$C_3$ alkyl groups; a $C_4$-$C_9$ cycloalkylcarbonyl group; a benzoyl group which may be substituted with a halogen atom, $C_1$-$C_4$ alkyl group or $C_1$-$C_4$ alkoxy group; a $C_2$-$C_7$ alkoxycarbonyl group; a $C_1$-$C_4$ alkylsufonyl group; or a carbamoyl group which may be substituted with a $C_1$-$C_4$ alkyl group and/or $C_1$-$C_4$ alkoxy group, and $Z^2$ represents a halogen atom such as chlorine atom and bromine atom, or a residue forming a symmetric or unsymmetric acid anhydride with $R^{52}$ when $R^{52}$ is an acyl group specified above, in the presence or absence of a base at a temperature from -30 to 120 °C.

7. A process for producing a diphenyl ether derivative represented by the following formula (Ic):

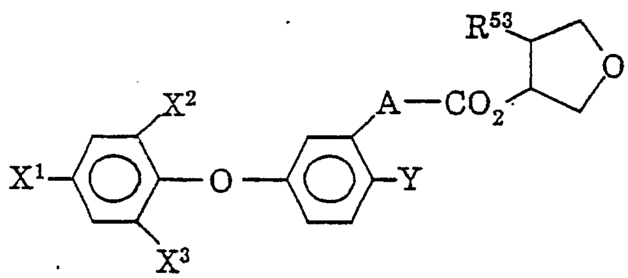

( I c)

wherein $R^{53}$ represents a halogen atom, and $X^1$, $X^2$, $X^3$, A and Y are the same as defined in claim 1, which comprises reacting a compound represented by the formula (Ia):

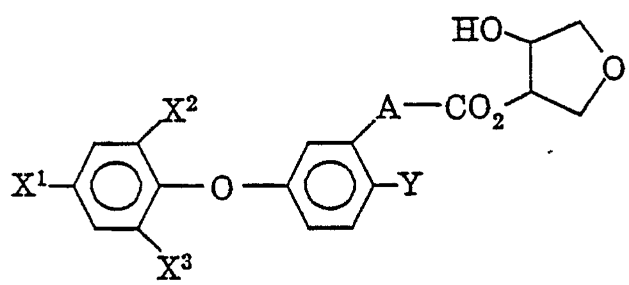

( I a)

wherein $X^1$, $X^2$, $X^3$, A and Y are the same as defined in claim 1, with a halogenating agent in the presence or absence of a base at a temperature from -30 to 60 °C.

8. A process for producing a diphenyl ether derivative represented by the following formula (Id):

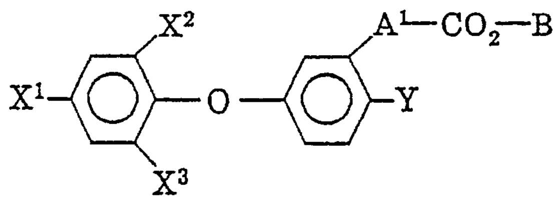

( I d)

wherein $A^1$ represents a group of the formula:

$-CO_2CH(R^1)-$

wherein $R^1$ is the same as defined in claim 1, a group of the formula:

$-C(R^{22})=NOCH(R^3)-$

wherein $R^{22}$ represents a hydrogen atom or a $C_1$-$C_4$ alkyl group which may be substituted with a $C_1$-$C_4$ alkoxy group, $C_1$-$C_4$ alkylthio group or a halogen atom and $R^3$ is the same as defined in claim 1, or a group of the formula:

$-C(=NOR^4)OCH(R^3)-$

wherein R3 and R4 are the same as defined in claim 1; and $X^1$, $X^2$, $X^3$, Y and B are the same as defined in claim 1,

which comprises reacting a compound of the following formula (III):

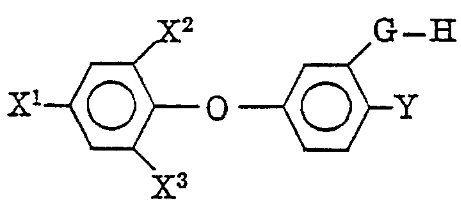

(III)

wherein $X^1$, $X^2$, $X^3$ and Y are the same as defined in claim 1, and G represents $-CO_2-$ when $A^1$ is $-CO_2CH(R^1)-$ or represents $-C(R^{22})=NO-$ or $-CONH(OR^4)-$ when $A^1$ is $-C(R^{22})=NOCH(R^3)-$ or $-C(=NOR^4)OCH(R^3)-$, with a compound of the formula (IV):

$Z^2-CH(R)CO_2B$ (IV)

wherein $Z^2$ represents a halogen atom, R is $R^1$ when G is $-CO_2-$ or $R^3$ when G is $C(R^{22})=NO-$ or $-CONH(OR^4)-$,

in the presence of a base at a temperature of -30 to 200 °C.

9. A process for producing a diphenyl ether derivative represented by the following formula (I e):

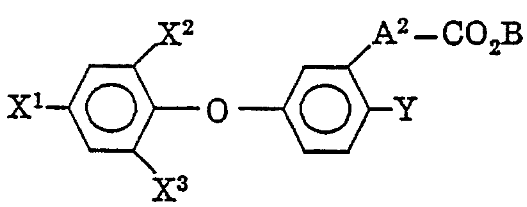

( I e)

wherein $X^1$, $X^2$, $X^3$, Y, and B are the same as defined in claim 1, and $A^2$ represents a group represented by the formula:

$-C(R^{23})=NOCH(R^3)-$

wherein $R^{23}$ represents a $C_1$-$C_4$ alkyl group which may be substituted with a $C_1$-$C_4$ alkoxy group, $C_1$-$C_4$ alkylthio group or halogen atom, and $R^3$ is the same as defined in claim 1, or a group represented by the formula:

$-C(=NOR^4)OCH(R^3)-$

wherein $R^3$ and $R^4$ are the same as defined in claim 1,

which comprises reacting a compound represented by the formula (V):

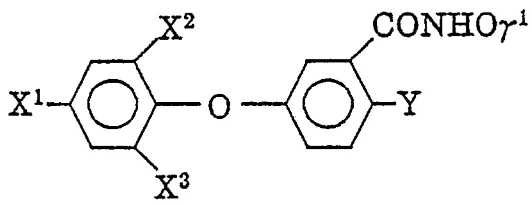

(V)

wherein $X^1$, $X^2$, $X^3$ and Y are the same as defined in claim 1, and $\gamma^1$ represents $-CH(R^3)CO_2B$ or $R^4$ wherein $R^3$, $R^4$ and B are the same as defined in claim 1,
with a compound of the formula (V):
$Z^3$-$\gamma^2$ (V)
wherein $Z^3$ represents a halogen atom or, a phenylsulfonyloxy or alkylsulfonyloxy group which may be substituted, and $\gamma^2$ represents $R^{23}$ when $\gamma^1$ is $-CH(R^3)CO_2B$ or represents $-CH(R^3)CO_2B$ when $\gamma^1$ is $R^4$ wherein $R^{23}$, $R^3$, $R^4$ and B are the same as defined above,
in the presence of a base at a temperature from -30 to 200 °C.